# DEMANDE DE BREVET EUROPEEN

(11) **EP 3 381 529 A1**
(43) Date de publication de la demande: **03.10.2018**
(21) Numéro de dépôt: 17305363.8
(22) Date de dépôt: 29.03.2017
(51) Int. Cl.: B01D 15/00, C12M 1/00, G01N 30/88, C12M 3/00

(54) **INSTALLATION DE TRAITEMENT DE LIQUIDE BIOLOGIQUE**

(71) Demandeur: EMD Millipore Corporation, Burlington, MA 01803 (US)
(72) Inventeur: CIROU, Sébastien, 67300 SCHILTIGHEIM (FR); ABOUAYAD EL IDRISSI, Christine, 67114 ESCHAU (FR)
(74) Mandataire: Santarelli

(57) **Abrégé**

L'invention concerne une installation de traitement de liquide biologique par chromatographie, s'étendant selon une direction longitudinale et comportant une vanne d'alimentation (20b), une pompe d'alimentation (30) en aval de la vanne, des organes d'instrumentation en aval de la pompe dont au moins une vanne de distribution (81a-c, 82a-c, 83a-c) et au moins un dispositif de mesure (78a-c, 85a-c, 86a-c) d'un paramètre physico-chimique du liquide, au moins une colonne de chromatographie (99a-c) en aval des organes d'instrumentation et des conduites connectant la vanne, la pompe, les organes d'instrumentation et la colonne, des organes d'instrumentation étant associés à la colonne de chromatographie et montés chacun sur au moins une plateforme dédiée de contrôle et de commande (80a-c), qui s'étend selon une direction verticale par rapport à la direction globalement longitudinale d'extension de ladite installation, et qui sont disposés sensiblement les uns au-dessus des autres sur ladite plateforme dédiée de contrôle et de commande.

## Description

### DOMAINE DE L'INVENTION

L'invention a trait aux installations de traitement de liquide biologique, en particulier mais non exclusivement pour purifier un liquide biopharmaceutique afin d'obtenir un produit tel que des anticorps monoclonaux, des vaccins ou des protéines recombinantes.

### ARRIERE PLAN TECHNOLOGIQUE

On sait que les liquides biopharmaceutiques sont en général obtenus par cultures en bioréacteur et qu'il faut ensuite les purifier pour atteindre les caractéristiques requises de pureté, de concentration, d'absence de virus, etc.

La purification est généralement réalisée au moyen d'une succession de traitements tels que la clarification pour éliminer les résidus de la culture en bioréacteur et un traitement de rétention virale suivi parfois d'un traitement de diafiltration et concentration par filtration tangentielle (TFF). Il existe encore, en matière de purification, d'autres opérations telles que la chromatographie (XMO).

Les traitements de purification sont réalisés essentiellement par des opérations de filtration dans un circuit aboutissant à un récipient de recueil du liquide traité. En entrée du circuit peuvent être raccordés plusieurs types de récipients contenant des liquides, comme le récipient source qui contient le produit à traiter, mais aussi les récipients contenant un liquide de nettoyage tel que de la soude (NaOH), un liquide de rinçage tel que de l'eau pure ou encore un liquide tampon tel qu'une solution saline.

En sortie du circuit peuvent être raccordés, en outre du récipient de recueil du liquide traité, divers autres récipients de recueil du liquide de nettoyage, de rinçage ou tampon ou encore de recueil des résidus.

Les traitements d'un liquide en production peuvent se succéder en séquence, le récipient de recueil du premier traitement devenant potentiellement le récipient source pour le traitement suivant, et ainsi de suite jusqu'à la réalisation du dernier traitement.

De façon traditionnelle, ces traitements sont réalisés dans des installations dédiées comportant des tuyauteries en acier inoxydable et d'autres composants tels que des cuves ou des boîtiers de filtres, qui nécessitent des opérations, avant et après le traitement proprement dit, relativement lourdes, en particulier de nettoyage après utilisation.

Depuis quelques années, ces traitements sont alternativement effectués dans des installations où les éléments en contact avec le liquide sont à usage unique. On connaît du document EP 2 585 187 une telle installation de traitement par chromatographie.

Cette installation comporte un dispositif formé d'une base, d'une presse à deux coquilles montées sur une face frontale de cette base, d'une poche enserrée entre cette presse et d'une plaque support fixé sur le côté de la base. Le dispositif a la forme d'un chariot monté sur quatre roulettes. Le dispositif est aussi pourvu, sur le bas, d'un caisson fermé et destiné à accueillir un ou plusieurs bacs si nécessaire. Un tableau de commande est agencé sur le haut de la face frontale du dispositif.

Sur l'avant des coquilles sont ménagées, en renfoncement, des gorges de conformation en regard pour former des conduites dans la poche et, sur l'arrière des coquilles sont implantées des instruments dont des capteurs de pression et des vannes à pincement qui sont configurées pour pincer les conduites de manière à interdire ou à autoriser le passage de liquide dans celles-ci. La poche est pourvue d'une pluralité de raccords pour liquide et d'un réseau de cheminement de liquide entre ces raccords dont les conduites susmentionnées. La plaque de support comporte deux têtes de fixation sur lesquelles une plateforme de contrôle est adaptée à être fixée pour y disposer des instruments pour le traitement du liquide biologique. Ces instruments peuvent par exemple être des capteurs mesurant le pH ou la conductivité.

Cette installation comporte en outre d'autres dispositifs, du type chariot, sur lesquels sont disposés des pompes, divers récipients contenant par exemple des liquides de rinçage, de nettoyage et/ou tampon, et/ou un produit d'élution ou prévus pour recevoir un recueil, une fraction ou un rebut ; d'autres instruments de mesure tels que capteur de présence produit, débulleur, capteurs de pression, de pH et/ou de conductivité ; un ou plusieurs éléments de filtration et une colonne de chromatographie ; tous ces éléments étant configurés pour être raccordés au circuit de la poche.

On connaît également du document EP 2 130 903 une installation qui comporte un premier chariot et un deuxième chariot qui peuvent soit être écartés soit être emboîtés l'un dans l'autre.

Chaque chariot se présente sous une forme globalement parallélépipédique et est monté sur roues afin de permettre son déplacement aisé au sein d'une zone de production.

Le premier chariot est ouvert sur un côté et vers le sol, son intérieur est évidé pour permettre l'emboîtement du deuxième chariot. Sur la partie supérieure du premier chariot se trouve un plateau support destiné à recevoir des éléments réutilisables du circuit et des moyens de support des éléments jetables. Parmi les éléments réutilisables portés par le plateau on trouve notamment une pompe de circulation, un premier capteur de pression et un panneau de commande pour piloter la pompe. Le plateau est positionné à une hauteur suffisante pour que le deuxième chariot puisse être glissé dessous et se positionner, au moins partiellement, sous la pompe de circulation lorsque les chariots sont emboités.

Le deuxième chariot présente un plateau pourvu d'une face supérieure sur laquelle sont positionnés des éléments jetables tels que des éléments de filtration et des éléments réutilisables tels qu'un deuxième capteur de pression. Ce deuxième chariot présente en outre des tiroirs de stockage destinés à loger des poches de recueil du liquide ou d'autres récipients tels que des poches d'échantillonnage ou de vidange.

Une conduite est reliée à une poche source contenant le liquide à traiter et comporte un élément adapté à coopérer avec la pompe pour faire circuler ce liquide vers les éléments filtrants, en passant par les capteurs de pression. Une autre conduite est reliée à l'élément de filtration pour faire circuler ce liquide traité vers la poche de recueil positionnée à l'intérieur d'un tiroir.

La diversité des traitements pouvant être effectués sur ce type d'installations est importante, notamment en fonction de la sélection par l'utilisateur du liquide à traiter et du degré de pureté à obtenir pour le liquide traité recueilli.

Cette grande diversité dans les traitements impose l'utilisation de nombreux éléments réutilisables et jetables différents d'un traitement à l'autre. En particulier, le nombre d'éléments de filtration et de moyens de contrôle et de commande associés à ces éléments et prévus pour mesurer et surveiller les paramètres du liquide traité peut augmenter. Par conséquent, l'agencement de ces éléments de filtration et moyens de contrôle et de commande nécessite d'être simple, commode et flexible.

### OBJET DE L'INVENTION

L'invention vise à fournir une installation permettant la mise en oeuvre de traitements de liquide biologique de manière simple, commode et économique.

Pour cela, l'invention concerne une installation de traitement de liquide biologique par chromatographie, s'étendant globalement selon une direction longitudinale et comportant :
- au moins une vanne d'alimentation en liquide biologique à traiter, configurée pour être raccordée au moins à un récipient d'alimentation en liquide biologique ;
- au moins une pompe d'alimentation disposée en aval de ladite au moins une vanne d'alimentation et raccordée à cette dernière ;
- une pluralité d'organes d'instrumentation disposés en aval de ladite au moins une pompe d'alimentation, dont au moins une vanne de distribution et au moins un dispositif de mesure d'un paramètre physico-chimique du liquide biologique, et qui sont raccordés à ladite au moins une pompe d'alimentation ;
- au moins une colonne de chromatographie disposée en aval de ladite pluralité d'organes d'instrumentation et directement associée et raccordée à au moins certains d'entre eux, et configurée pour être alimentée en liquide biologique par ladite au moins une pompe d'alimentation ; et
- une pluralité de conduites à usage unique configurées pour être connectées à ladite au moins une vanne d'alimentation, à ladite au moins une pompe d'alimentation, à ladite pluralité d'organes d'instrumentation et à ladite au moins une colonne de chromatographie, de sorte à former au moins une ligne d'alimentation de liquide biologique à traiter d'un circuit de traitement de ladite installation ;
caractérisée en ce que lesdits organes d'instrumentation associés à ladite au moins une colonne de chromatographie sont montés chacun sur au moins une plateforme dédiée de contrôle et de commande, qui s'étend selon une direction verticale par rapport à la direction globalement longitudinale d'extension de ladite installation, et sont disposés sensiblement les uns au-dessus des autres sur ladite plateforme dédiée de contrôle et de commande.

L'installation selon l'invention présente un agencement qui assure en premier lieu une mise en place centralisée des organes d'instrumentation directement associés à la colonne de chromatographie en positionnant ces derniers sur une même et unique plateforme individuelle de contrôle et de commande.

Au surplus, un tel agencement sensiblement superposé de ces organes d'instrumentation sur la plateforme verticale offre une compacité particulièrement avantageuse en comparaison aux installations où les organes d'instrumentation sont plutôt éparpillés.

Cette compacité est d'autant plus avantageuse dans le contexte de l'invention puisqu'elle permet de réduire l'empreinte au sol de l'installation, qui nécessite d'être implantée dans des salles dites blanches, aux contraintes sanitaires particulièrement sévères.

Par ailleurs, un tel agencement sensiblement superposé des organes d'instrumentation sur la plateforme verticale facilite l'accès à ces organes d'instrumentation et donc leur raccordement notamment à la colonne de chromatographie et à la pompe d'alimentation. Il est aussi particulièrement aisé de remplacer la plateforme verticale et ses organes d'instrumentation par une autre plateforme, voire de remplacer seulement un des organes d'instrumentation sur cette plateforme.

Un tel agencement offre ainsi un montage/démontage simple et rapide de l'installation en facilitant les branchements des conduites souples, voire en limitant les croisements de ces conduites.

Par ailleurs, un tel agencement est particulièrement reproductible en cas de multiplication du nombre de colonnes de chromatographie et donc du nombre d'organes d'instrumentation associés, tout en conservant une grande flexibilité, un accès et tout en permettant en outre de réduire de manière significative la longueur des conduites jetables du circuit de traitement. Ainsi, on minimise le volume de liquide interne au circuit de l'installation. On notera que dans ce cas, l'installation comporte généralement au moins deux pompes.

Il ressort donc de ce qui précède que l'installation pour traitement de liquide biologique selon l'invention est particulièrement simple, commode et économique.

Selon d'autres caractéristiques préférées, simples, commodes et économiques de l'installation selon l'invention :
- ladite au moins une plateforme de contrôle et de commande comporte plusieurs vannes de distribution et un ou plusieurs dispositifs de mesure choisis parmi un capteur de conductivité et/ou un capteur de pH et/ou un capteur de présence d'air ;
- lesdites vannes de distribution sont pourvues chacune d'un corps de vanne et d'une tête de vanne qui s'étend depuis ledit corps et qui est prévue pour recevoir au moins des portions de conduites de ladite ligne d'alimentation, et ladite au moins une plateforme de contrôle et de commande comporte un bloc support qui s'étend verticalement, dans lequel sont logés lesdits corps de vanne et desquels saillent latéralement lesdites têtes de vanne, ainsi qu'une plaque support fixée sur ledit bloc support et présentant un bras qui saille dudit bloc support et sur lequel est monté ledit au moins un dispositif de mesure ;
- l'installation comporte plusieurs colonnes de chromatographie disposées en aval de ladite au moins une plateforme de contrôle et de commande, et qui s'étendent selon une direction globalement transversale à ladite direction globalement longitudinale d'extension de ladite l'installation ; lesdites colonnes de chromatographie pouvant être de préférence agencées en triangle si elles sont au moins au nombre de trois ;

- l'installation comporte plusieurs plateformes dédiées de contrôle et de commande, chacune étant individuellement associée à une desdites colonnes de chromatographie, lesdites plateformes dédiées de contrôle et de commande étant disposées selon la même direction globalement transversale que lesdites colonnes de chromatographie ;
- lesdites plateformes de contrôle et de commande sont montées sur un premier chariot et lesdites colonnes de chromatographie sont montées sur un deuxième chariot configuré pour être accolé et/ou partiellement emboîté avec ledit premier chariot ;
- ledit premier chariot comporte un premier châssis, au moins une armoire de distribution électrique et pneumatique montée sur ledit châssis et sur laquelle est disposée ladite au moins une vanne d'alimentation, au moins un espace d'accueil formé dans ledit châssis et prévu pour recevoir des récipients de récupération de liquides, et au moins un plateau support monté saillant sur ledit châssis et sur lequel sont disposées ladite au moins une pompe d'alimentation et lesdites plateformes de contrôle et de commande, et ledit deuxième chariot comporte un deuxième châssis pourvu d'une planche support prévue pour recevoir lesdites colonnes de chromatographie et configurée pour venir au moins partiellement en emboîtement sous ledit plateau dudit premier chariot ;
- l'installation comporte en outre des supports d'instrumentation additionnels disposés en aval desdites colonnes de chromatographie et sur lesquels sont montés un ou plusieurs dispositifs de mesure additionnels choisis parmi un capteur de conductivité, et/ou un capteur de pH et/ou un capteur de rayonnement UV ;
- les supports d'instrumentation additionnels peuvent être individuellement associés à une desdites colonnes de chromatographie, et/ou disposés selon la même direction globalement transversale que lesdites colonnes de chromatographie et que lesdites plateformes dédiées de contrôle et de commande ;
- lesdits supports d'instrumentation additionnels sont montés sur un troisième chariot configuré pour être accolé et/ou partiellement emboîté ledit premier chariot et/ou avec ledit deuxième chariot ;
- l'installation comporte en outre une pluralité d'organes d'instrumentation supplémentaires disposés en aval desdites colonnes de chromatographie, dont au moins une vanne de sortie et au moins un dispositif de mesure supplémentaire choisis parmi un capteur de pression et/ou un spectrophotomètre, lesdits organes d'instrumentation supplémentaires étant montés sur ledit troisième chariot, en aval desdits supports d'instrumentation additionnels ;
- ledit troisième chariot comporte un troisième châssis, sensiblement en forme de U inversé, sur lequel sont montés au moins lesdits supports d'instrumentation additionnels à une extrémité libre dudit troisième châssis, et qui est configuré pour être accolé audit premier châssis dudit premier chariot par une extrémité opposée à son extrémité libre, et pour recevoir à emboîtement, dans un espace formé par le U inversé entre son extrémité opposée et son extrémité libre, ledit deuxième châssis dudit deuxième chariot pourvu desdites colonnes de chromatographie ;
- ladite au moins une vanne d'alimentation et/ou ladite au moins une vanne de distribution sont des vannes à trois voies, dont deux entrées et une sortie ou une entrée et deux sorties, et de préférence une vanne pourvue d'une tête présentant deux gorges de réception de portions de dites conduites, et d'un mécanisme à pincement configuré pour autoriser ou interdire la circulation dudit liquide biologique dans lesdites portions de dites conduites reçues dans les deux dites gorges ;
- l'installation comporte en outre un récipient de réserve disposé en aval de ladite au moins une colonne de chromatographie et raccordé à cette dernière, ledit récipient de réserve étant prévu pour recevoir un produit tampon utilisé pour le nettoyage de ladite au moins une colonne de chromatographie après traitement dudit liquide biologique, et une autre vanne d'alimentation disposée en aval dudit récipient de réserve et en amont de ladite au moins une pompe d'alimentation et raccordée à ceux-ci, ladite autre vanne d'alimentation étant configurée pour réintroduire, via ladite au moins une pompe d'alimentation, ledit produit tampon sur ladite ligne d'alimentation en tant que liquide à traiter dans ladite au moins une colonne de chromatographie ;
- l'installation comporte en outre une pluralité de vannes d'entrée configurées pour être raccordées à des récipient de produits dits tampon et pour alimenter ledit circuit de traitement en vue de la préparation et/ou du nettoyage et/ou de l'élution et/ou de la régénération de ladite au moins une colonne de chromatographie, et au moins une pompe additionnelle disposée en aval desdites vannes d'entrée et en amont de ladite au moins une colonne de chromatographie et raccordée à ces dernières ; des conduites de ladite pluralité de conduites à usage unique étant configurées pour être connectées auxdites vannes d'entrée et à ladite pompe additionnelle, de sorte à former au moins une ligne additionnelle, qui s'étend sensiblement longitudinalement et en parallèle de ladite ligne d'alimentation, depuis respectivement lesdites vannes d'entrée et ladite au moins une vanne d'alimentation, vers ladite au moins une plateforme dédiée de contrôle et de commande ; et/ou
- l'installation comporte en outre d'autres organes d'instrumentation, dont, sur ladite ligne d'alimentation :
   - un capteur de présence produit disposé en amont de ladite au moins une vanne d'alimentation ; et/ou
   - une vanne deux voies d'isolation et/ou un débitmètre disposés entre ladite au moins une vanne d'alimentation et ladite au moins une pompe d'alimentation ; et/ou
   - un capteur de pression avec ou sans sécurité, et/ou au moins une vanne de drain, et/ou au moins deux vannes de débulleur et un débulleur raccordé à chacune de ces vannes de débulleur et/ou au moins une vanne de filtre et un filtre raccordé à ladite au moins une vanne de filtre, disposés entre ladite au moins une pompe d'alimentation et ladite au moins une plateforme de contrôle et de commande ; et/ou
sur ladite ligne additionnelle :
- une vanne deux voies d'isolation et/ou un débitmètre disposés entre lesdites vannes d'entrée et ladite au moins une pompe additionnelle ; et/ou
- au moins un capteur de pression avec ou sans sécurité, et/ou au moins une vanne de drain, et/ou au moins deux vannes de débulleur et un débulleur raccordé à chacune de ces vannes de débulleur, disposés entre ladite pompe additionnelle et ladite au moins une plateforme de contrôle et de commande.

### BREVE DESCRIPTION DES DESSINS

L'exposé de l'invention sera maintenant poursuivi par la description d'exemples de réalisation, donnée ci-après à titre illustratif et non limitatif, en référence aux dessins annexés sur lesquels :
- les figures 1 à 3 représentent, schématiquement en perspective, une installation de traitement conforme à l'invention, selon différents angles de vue et dans différentes configurations d'assemblage ;
- les figures 4 à 8 sont des vues, respectivement de face, de trois-quarts arrière, de côtés et de dessus, de l'installation dans sa configuration illustrée à la figure 3 ;
- les figures 9 à 11 représentent, schématiquement en perspective, une plateforme de contrôle et de commande de l'installation illustrée sur les figures précédentes, selon différents angles de vue ; et
- la figure 12 est une vue schématique d'un circuit de traitement par chromatographie de liquide biologique, mis en oeuvre dans l'installation illustrée sur les figures 1 à 8.

### DESCRIPTION DETAILLEE D'AU MOINS UN EXEMPLE DE REALISATION

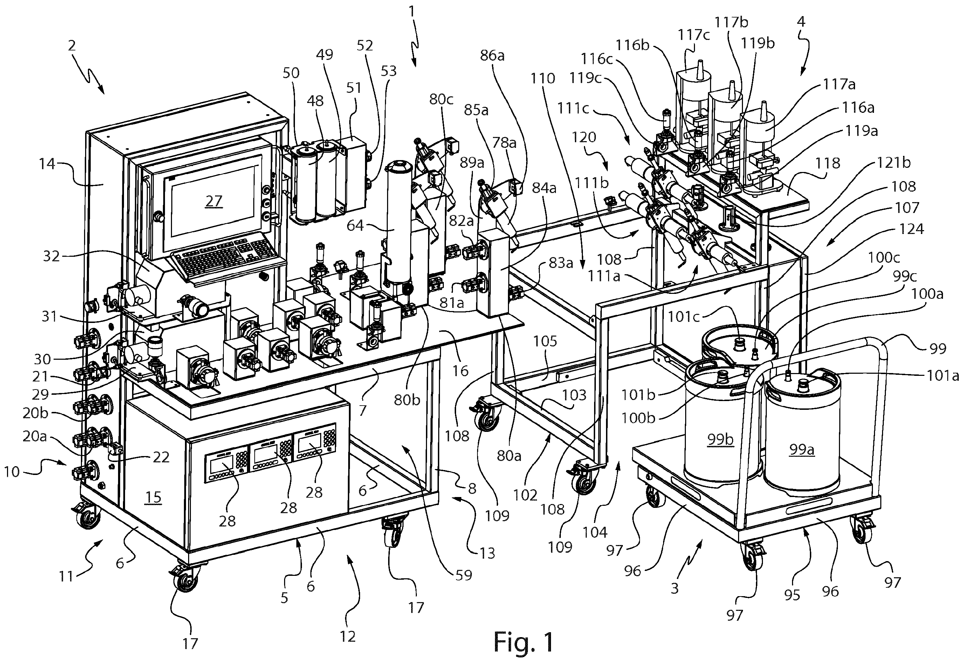

Les figures 1 à 3 illustrent une installation 1 de traitement par chromatographie, dans différentes configurations d'assemblage.

L'installation 1 comporte ici un premier chariot 2, un deuxième chariot 3 ainsi qu'un troisième chariot 4, lesquels sont configurés pour être accolés et au moins partiellement emboîtés les uns dans les autres.

Sur la figure 1, les premier, deuxième et troisième chariots 2 à 4 sont séparés et à distance les uns des autres. Les premier et troisième chariots 2 et 4 sont agencés selon une même direction longitudinale tandis que le deuxième chariot 3 se situe, de manière décalée, devant le troisième chariot 4.

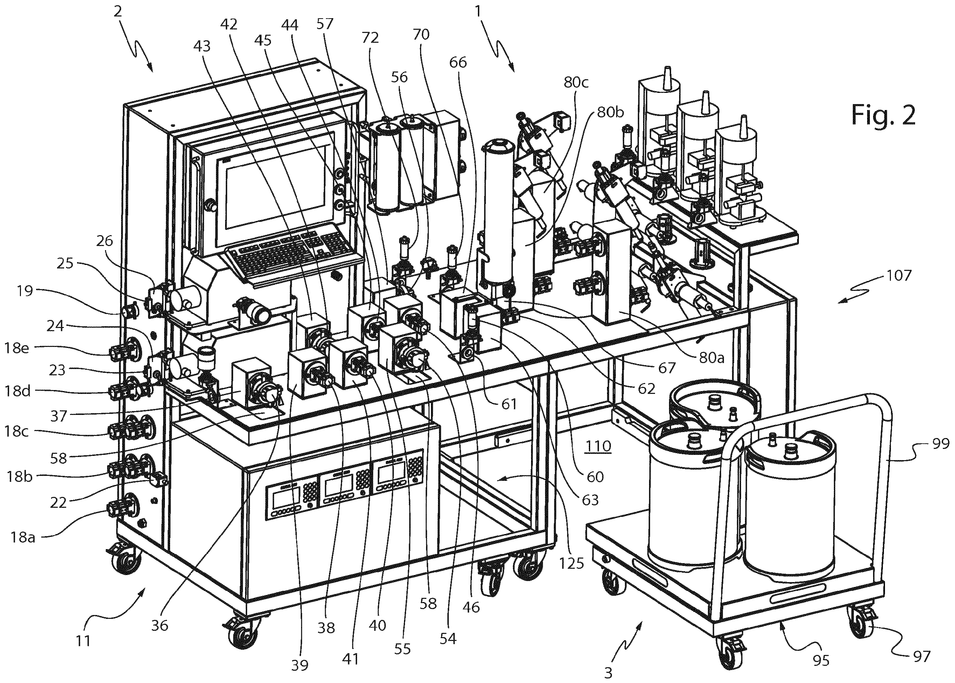

Sur la figure 2, les premier et troisième chariots 2 et 4 sont accolés et emboîtés selon la direction longitudinale d'extension de l'installation 1, tandis que le deuxième chariot 3 se situe toujours de manière décalée devant le troisième chariot 4.

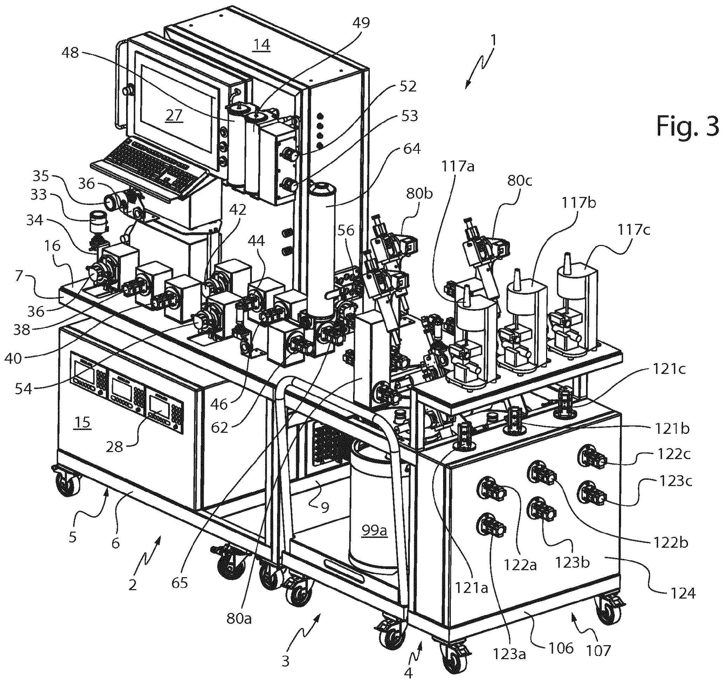

Sur la figure 3, le deuxième chariot 3 est partiellement emboîté dans le troisième chariot 4 et situé à proximité immédiate du premier chariot 2 de sorte que ce deuxième chariot 3 est aussi partiellement emboîté et accolé avec le premier chariot 2.

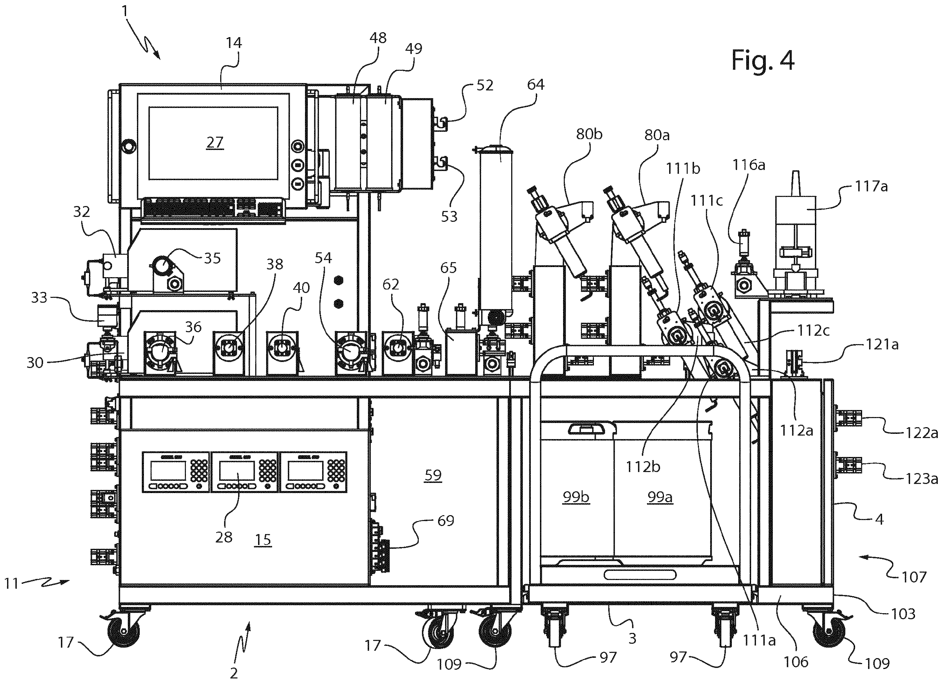

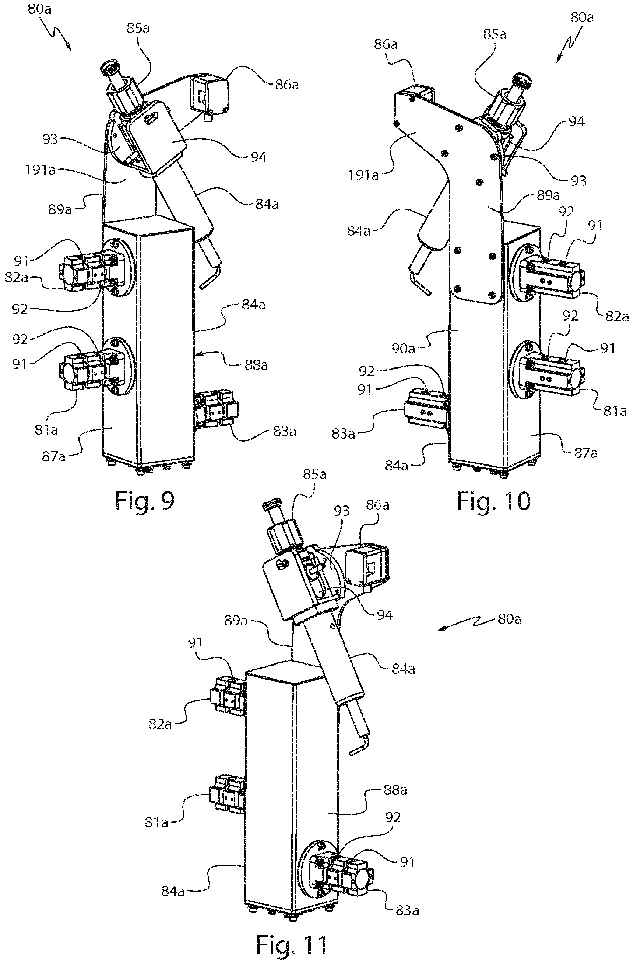

On va maintenant décrire en détail la structure de chacun des premier, deuxième et troisième chariots 2 à 4 et les éléments qu'ils portent, en références aux figures 1 à 11, où les figures 4 à 8 sont des vues, respectivement de face, de trois-quarts arrière, de côtés et de dessus, de l'installation 1 avec ses premier, deuxième et troisième chariots 2 à 4 assemblés, et où les figures 9 à 11 représentent une plateforme de contrôle et de commande de l'installation.

Le premier chariot 2 est pourvu d'un premier châssis 5 métallique présentant un premier cadre inférieur 6 ici sensiblement rectangulaire, un premier cadre supérieur 7 ici hexagonal, en forme sensiblement de L, et situé partiellement en regard et à distance du premier cadre inférieur 6, et plusieurs montants verticaux 8 assujettis aux premiers cadres inférieur et supérieur 6 et 7 de sorte à former un premier châssis 5 rigide.

Le premier cadre inférieur 6 est ici monté sur quatre roulettes pour faciliter son déplacement.

Chacun des premiers cadres inférieur et supérieur 6 et 7 est formé de barres longitudinales, appelées aussi longerons, s'étendant selon une direction longitudinale, et de barres transversales, appelées aussi traverses, s'étendant selon une direction transversale, ici sensiblement orthogonale à la direction longitudinale. Le premier cadre inférieur 6 est en outre formé d'une traverse intermédiaire 9 disposée entre ses deux barres transversales et assujettie à ses deux barres longitudinales.

Le premier chariot 2 porte une armoire principale de distribution électrique et pneumatique 14 montée sur le premier cadre inférieur 6, sur l'arrière 10 et sur un premier côté 11 du premier chariot 2, une armoire secondaire 15 montée aussi sur le premier cadre inférieur 6, mais plutôt sur l'avant 12 et sur le premier côté 11 du premier chariot 2. L'armoire secondaire 15 est ainsi disposée devant l'armoire principale 14.

L'armoire principale 14 s'étend globalement verticalement, ce qui signifie qu'elle est plus haute que longue, depuis le premier cadre inférieur 6 et au-delà du premier cadre supérieur 7, tandis que l'armoire secondaire 15 s'étend globalement longitudinalement, ce qui signifie qu'elle est plus longue que haute, entre les premiers cadres inférieur et supérieur 6 et 7, depuis la barre transversale située sur le premier côté 11 du premier cadre inférieur 6 jusqu'à sa traverse intermédiaire 9.

Le premier chariot 2 porte en outre un plateau support 16 monté sur le premier cadre supérieur 7 et saillant de ce dernier sur un deuxième côté 13 du premier chariot 2, opposé à son premier côté 11 ; de sorte qu'une portion du plateau support 16 se trouve en porte-à-faux vis-à-vis du premier châssis 5.

On notera que le plateau support 16 présente ici un contour sensiblement en forme de L pour se conformer au contour du premier cadre supérieur 7, et est pourvu d'un décrochement pour permettre le passage de l'armoire principale 14.

Le plateau support 16 est configuré pour recevoir des éléments réutilisables de l'installation de traitement 1 et des moyens de support à la fois pour ces éléments réutilisables mais aussi pour des éléments jetables de cette installation 1.

En particulier ici, l'installation 1 comporte une pluralité de vannes d'alimentations disposées sur le premier côté 11 du premier chariot 2 et logées partiellement dans une face latérale de l'armoire principale 14, selon deux rangées distinctes.

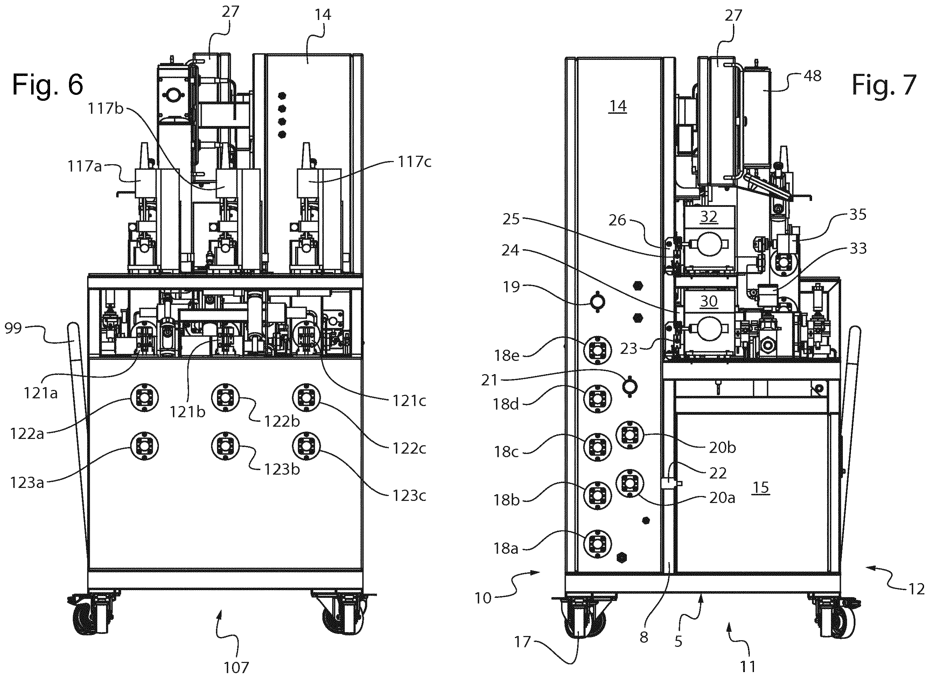

La rangée située la plus sur l'arrière 10 du premier chariot 2 présente cinq vannes trois voies 18a à 18e superposées et une vanne deux voies d'isolation 19 située au-dessus des cinq vannes trois voies 18a à 18e; tandis que l'autre rangée présente deux vannes trois voies 20a et 20b superposées et une vanne deux voies d'isolation 21 située au-dessus des deux vannes trois voies 20a et 20b (figures 2 et 7). On notera que les vannes d'isolation 19 et 21 sont ici configurées pour fonctionner soit en tout ou rien, soit de manière proportionnelle.

On notera que chacune des vannes 18 à 20 présentent un corps logé dans l'armoire principale 14 et une tête prolongeant le corps et saillant de l'armoire principale 14 sur le premier côté 11 du premier chariot 2.

Au surplus, chacune des vannes 18 à 20 est configurée pour être raccordées par des conduites souples jetables à des récipients présentant des produits déterminés.

En particulier, les vannes 20a à 20b sont des vannes d'alimentation configurées pour être raccordées à des récipients de liquide biologique à traiter par chromatographie et forment le début d'une ligne d'alimentation d'un circuit de traitement par chromatographie de l'installation 1.

Pour ce faire, la vanne 20a est ici configurée pour être raccordée à un récipient de produit tampon d'équilibration et à un récipient dit de réserve de liquide à retraiter, et la vanne 20b est ici configurée pour être raccordée à la vanne 20a et à un récipient de liquide biologique à traiter.

La vanne 21 est quant à elle une vanne proportionnelle ici configurée pour être raccordée à la vanne 20b ainsi qu'au reste de la ligne d'alimentation se prolongeant en aval des vannes 20a, 20b et 21.

Au surplus, les vannes 18a à 18e sont des vannes d'entrée configurées pour être raccordées à des récipient de produits dits tampon et pour alimenter un circuit de traitement de l'installation en vue de la préparation et/ou du nettoyage et/ou de l'élution et/ou de la régénération de colonnes de chromatographie et forment le début d'une ligne additionnelle du circuit de traitement par chromatographie de l'installation 1.

Pour ce faire, la vanne 18a est ici configurée pour être raccordée à un récipient de produit tampon d'équilibration et à un récipient de premier produit tampon de lavage, la vanne 18b est ici configurée pour être raccordée à la vanne 18a et à un récipient de deuxième produit tampon de lavage, la vanne 18c est ici configurée pour être raccordée à la vanne 18b et à un récipient de produit tampon d'élution, la vanne 18d est ici configurée pour être raccordée à la vanne 18c et à un récipient de produit de nettoyage, et la vanne 18e est ici configurée pour être raccordée à la vanne 18d et à un récipient de produit tampon de régénération.

La vanne d'isolation 19 est quant à elle une vanne de distribution ici configurée pour être raccordée à la vanne 18e ainsi qu'au reste de la ligne additionnelle se prolongeant en aval des vannes 18a à 18e et 19.

L'installation 1 comporte un capteur de présence d'air 22 ici fixé sur un montant vertical 8 sur le premier côté 11 du premier chariot 2 et à proximité immédiate de la vanne d'alimentation 20a. Le capteur de présence d'air 22 est ici configuré pour être raccordé sur une portion de conduite entre le récipient de liquide biologique et la vanne d'alimentation 20b et permet de détecter et/ou l'absence de produit.

L'installation 1 comporte un premier débitmètre 23 monté via une console de fixation 24 sur un montant vertical 8 sur le premier côté 11 du premier chariot 2. Ce premier débitmètre 23 est configuré pour être raccordé sur la ligne d'alimentation, en amont, à la vanne d'isolation 21.

L'installation 1 comporte un second débitmètre 25 monté via une console de fixation 26 sur le même montant vertical 8 sur le premier côté 11 du premier chariot 2. Ce second débitmètre 25 est configuré pour être raccordé sur la ligne additionnelle, en amont, à la vanne d'isolation 19.

L'installation 1 comporte un système principal de traitement de données 27 fixé sur l'armoire principale 14 et formé ici par un ordinateur muni d'un clavier et d'un écran qui sont accessibles depuis l'avant 12 du premier chariot 2, ainsi qu'un système secondaire de traitement de données 28 formé ici par plusieurs tableaux de commande et de contrôle logés dans l'armoire secondaire et accessible aussi depuis l'avant 12 du premier chariot 2.

On notera que l'armoire principale 14 est pourvue de boutons de commande 68 sur une face de fond à l'arrière 10 du premier chariot 2 et de connecteurs pneumatiques et électriques 69 ménagés sur une face latérale de cette armoire 14, qui est opposée à la face latérale d'où saillent les vannes 18a à 18e, 19, 20a, 20b et 21.

L'installation 1 comporte un premier support de pompe 29 fixé sur le devant de l'armoire principale 14 et sur lequel est montée une pompe d'alimentation 30, ainsi qu'un second support de pompe 31 fixé aussi sur le devant de l'armoire principale 14 et sur lequel est montée une pompe additionnelle 32.

La pompe d'alimentation 30 est ici configurée pour être raccordée sur la ligne d'alimentation en aval des vannes d'alimentation 20a et 20b et d'isolation21 et est pourvue d'une tête de pompe disposée en regard du premier débitmètre 23 et raccordée à ce dernier ; tandis que la pompe additionnelle 32 est ici configurée pour être raccordée sur la ligne additionnelle en aval des vannes d'entrée 18a à 18e et d'isolation19 et est pourvue d'une tête de pompe disposée en regard du second débitmètre 25 et raccordée à ce dernier. Ces pompes 30 et 32 sont par exemple du type à diaphragme et sont configurées pour faire circuler les produits présents dans les récipients connectés aux vannes 18a à 18e, 20a et 20b en fonction de l'état de ces dernières.

L'installation 1 comporte un premier capteur de pression à organe de sécurité 33 monté, via une console de fixation 34, sur le plateau support 16, sur le premier côté 11 du premier chariot 2 ; ainsi qu'un second capteur de pression à organe de sécurité 35 monté, via une console de fixation 36, sur le second support de pompe 31.

Le premier capteur de pression à organe de sécurité 33 est ici configuré pour être raccordé sur la ligne d'alimentation en aval de la pompe d'alimentation 30 ; tandis que le second capteur de pression à organe de sécurité 35 est ici configuré pour être raccordé sur la ligne additionnelle en aval de la pompe additionnelle 32.

Les premier et second capteurs de pression à organe de sécurité 33 et 35 sont chacun configurés pour mesurer la pression du liquide circulant dans la ligne respective du circuit de traitement et pour arrêter la pompe respective au-delà d'une certaine valeur seuil de pression.

L'installation 1 comporte une première vanne de drain 36 pourvue d'un corps logé dans un bloc support 37 monté sur le plateau support 16, sur le côté de la pompe d'alimentation 30 vers l'avant 12 du premier chariot 2, et d'une tête prolongeant le corps et saillant du bloc support. Cette première vanne de drain 36 est ici configurée pour être raccordée sur la ligne d'alimentation, en amont, à la pompe d'alimentation 30 et, en aval, notamment à un récipient de rebut.

L'installation 1 comporte une première vanne de débulleur 38 pourvue d'un corps logé dans un bloc support 39 monté sur le plateau support 16, à côté de la première vanne de drain 36 vers le deuxième côté 13 du premier chariot 2, et d'une tête prolongeant le corps et saillant du bloc support. Cette première vanne de débulleur 38 est ici configurée pour être raccordée, sur la ligne d'alimentation, en amont, à la première vanne de drain 36 et, en aval, notamment à un premier débulleur 48 (voir ci-après).

L'installation 1 comporte une deuxième vanne de débulleur 40 pourvue d'un corps logé dans un bloc support 41 monté sur le plateau support 16, à côté de la première vanne de débulleur 38 vers le deuxième côté 13 du premier chariot 2, et d'une tête prolongeant le corps et saillant du bloc support. Cette deuxième vanne de débulleur 40 est ici configurée pour être raccordée, sur la ligne d'alimentation, en amont, à la première vanne de débulleur 38 et au premier débulleur 48.

Ce premier débulleur 48 est monté, via une console de fixation 50, sur un montant vertical 8 du premier châssis 5, sur un côté de l'armoire principale 14 opposé aux vannes d'alimentation et d'entrée, et plutôt sur le deuxième côté 13 du premier chariot. Ce premier débulleur 48 est ici configuré pour être raccordé, en entrée, à la première vanne de débulleur 38, en sortie principale, à la deuxième vanne de débulleur 40 et, en sortie secondaire, à une première vanne d'évent 52.

Cette première vanne d'évent 52 est pourvue d'un corps logé dans un bloc support 51 fixé sur la console de fixation 50, et d'une tête prolongeant le corps et saillant du bloc support. Cette première vanne d'évent 52 est ici configurée pour déboucher à l'atmosphère.

L'installation 1 comporte une deuxième vanne de drain 42 pourvue d'un corps logé dans un bloc support 43 monté sur le plateau support 16, en arrière de la pompe d'alimentation 30 et vers le deuxième côté 13 du premier chariot 2, et d'une tête prolongeant le corps et saillant du bloc support. Cette deuxième vanne de drain 42 est ici configurée pour être raccordée sur la ligne additionnelle, en amont, à la pompe additionnelle 32 et, en aval, notamment à un récipient de rebut.

L'installation 1 comporte une troisième vanne de débulleur 44 pourvue d'un corps logé dans un bloc support 45 monté sur le plateau support 16, à côté de la deuxième vanne de drain 42 vers le deuxième côté 13 du premier chariot 2, et d'une tête prolongeant le corps et saillant du bloc support. Cette troisième vanne de débulleur 44 est ici configurée pour être raccordée, sur la ligne additionnelle, en amont, à la deuxième vanne de drain 42 et, en aval, notamment à un second débulleur 49 (voir ci-après).

L'installation 1 comporte une quatrième vanne de débulleur 46 pourvue d'un corps logé dans un bloc support 47 monté sur le plateau support 16, à côté de la troisième vanne de débulleur 44 vers le deuxième côté 13 du premier chariot 2, et d'une tête prolongeant le corps et saillant du bloc support. Cette quatrième vanne de débulleur 46 est ici configurée pour être raccordée, sur la ligne additionnelle, en aval, à la troisième vanne de débulleur 44 et au second débulleur 49.

Ce second débulleur 49 est monté, via la console de fixation 50, sur le montant vertical 8 du premier châssis 5, sur le côté de l'armoire principale 14 opposé aux vannes d'alimentation et d'entrée, et plutôt sur le deuxième côté 13 du premier chariot 2. Ce second débulleur 48 est ici configuré pour être raccordé, en entrée, à la troisième vanne de débulleur 44, en sortie principale, à la quatrième vanne de débulleur 46 et, en sortie secondaire, à une seconde vanne d'évent 53.

Cette seconde vanne d'évent 53 est pourvue d'un corps logé dans le bloc support 51 fixé sur la console de fixation 50, et d'une tête prolongeant le corps et saillant du bloc support. Cette seconde vanne d'évent 53 est ici configurée pour déboucher à l'atmosphère.

L'installation 1 comporte une troisième vanne de drain 54 pourvue d'un corps logé dans un bloc support 55 monté sur le plateau support 16, à côté de la deuxième vanne de débulleur 40 vers le deuxième côté 13 du premier chariot 2, et d'une tête prolongeant le corps et saillant du bloc support. Cette troisième vanne de drain 54 est ici configurée pour être raccordée sur la ligne d'alimentation, en amont, à la deuxième vanne de débulleur 40 et, en aval, notamment à un récipient de rebut.

L'installation 1 comporte en outre une quatrième vanne de drain 56 pourvue d'un corps logé dans un bloc support 57 monté sur le plateau support 16, à côté de la quatrième vanne de débulleur 46 vers l'arrière 10 du premier chariot 2, et d'une tête prolongeant le corps et saillant du bloc support. Cette quatrième vanne de drain 56 est ici configurée pour être raccordée sur la ligne additionnelle, en amont, à la quatrième vanne de débulleur 46 et, en aval, notamment à un récipient de rebut.

On notera que des fenêtres 58 sont ménagées dans le plateau support 16 sous les vannes de drain 36, 42, 54 et 56 pour permettre de passer des portions de conduites du circuit de traitement vers les récipients de rebut qui peuvent être logés par exemple dans un espace d'accueil 59 ménagé entre les premiers cadres inférieur et supérieur 6 et 7 et entre l'armoire secondaire 15 et le deuxième côté 13 du premier châssis 5.

L'installation 1 comporte un troisième capteur de pression 60, ici sans sécurité, monté via une patte de fixation 61 ici en forme de S sur le plateau support 16, à côté de la troisième vanne de drain 54 vers le deuxième côté 13 du premier chariot 2. Le troisième capteur de pression 60 est ici configuré pour être raccordé sur la ligne d'alimentation en aval de la troisième vanne de drain 54.

L'installation 1 comporte une première vanne de filtre 62 pourvue d'un corps logé dans un bloc support 63 monté sur le plateau support 16, à côté du troisième capteur de pression 60 vers le deuxième côté 13 du premier chariot 2. La première vanne de filtre 62 est ici configurée pour être raccordée sur la ligne d'alimentation, en amont, au troisième capteur de pression 60 et, en aval, à un filtre 64 (voir ci-après).

L'installation 1 comporte une seconde vanne de filtre 65 pourvue d'un corps logé dans un bloc support 66 monté sur le plateau support 16, à côté de la première vanne de filtre 62 vers l'arrière 10 du premier chariot 2. La seconde vanne de filtre 65 est ici configurée pour être raccordée sur la ligne d'alimentation, en amont, au filtre 64 et la première vanne de filtre 62.

Ce filtre 64 est monté, via une console de fixation 67 assujettie mécaniquement sur le haut des blocs supports 63 et 66 respectivement des première et deuxième vannes de filtre 62 et 65, sensiblement au-dessus de ces première et deuxième vannes de filtre 62 et 65. Ce filtre 64 est ici configuré pour être raccordé, en entrée, à la première vanne de filtre 62, et en sortie, à la deuxième vanne de filtre 65.

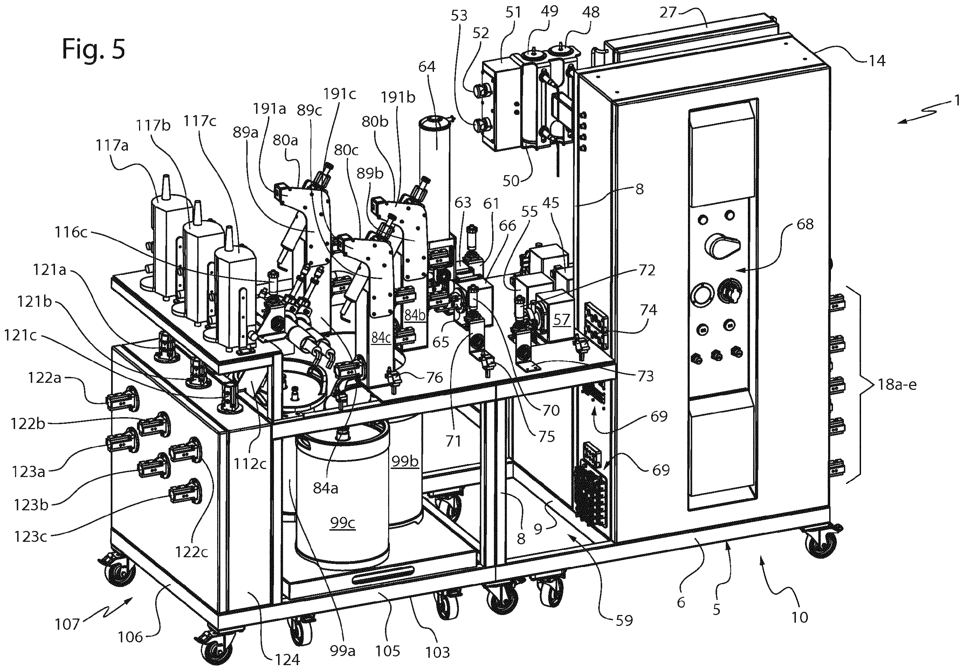

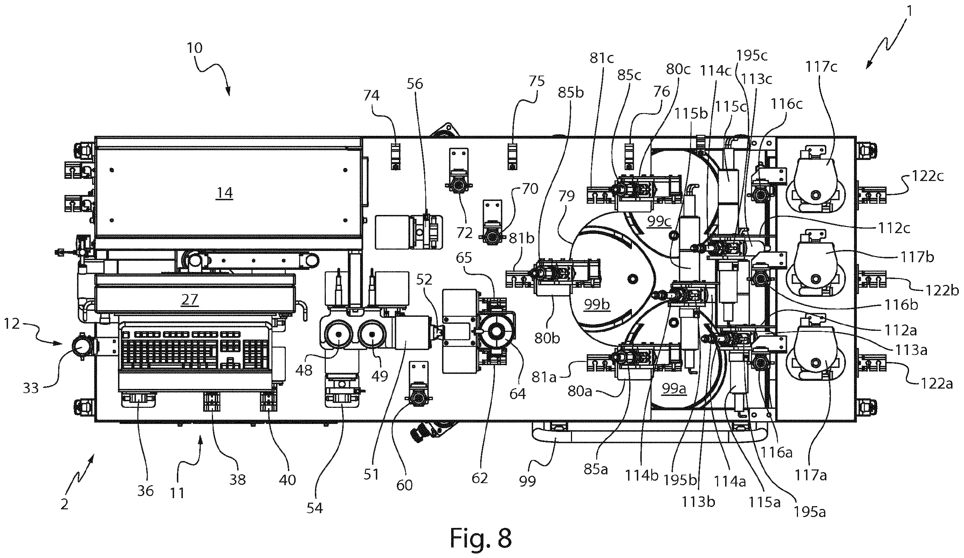

L'installation 1 comporte un quatrième capteur de pression 70, ici sans sécurité, monté via une patte de fixation 71 ici en forme de S sur le plateau support 16, à proximité de la deuxième vanne de filtre 65 vers l'arrière 10 du premier chariot 2 ; ainsi qu'un cinquième capteur de pression 72 ici sans sécurité et monté, via une console de fixation 73 ici en forme de S, sur le plateau support 16 à proximité de la quatrième vanne de drain 56 et du quatrième capteur de pression 70, vers l'arrière 10 du premier chariot 2 (figures 5 et 8).

Le quatrième capteur de pression 70 est ici configuré pour être raccordé sur la ligne d'alimentation, en amont, à la deuxième vanne de filtre 65 ; tandis que le cinquième capteur de pression 72 est ici configuré pour être raccordé sur la ligne additionnelle, en amont, à la quatrième vanne de drain 56.

L'installation 1 comporte en outre un chemin de câbles formé par des colliers 74 à 76 prévus pour le cheminement de câbles électriques et pneumatiques (non représentés) qui courent le long d'un bord arrière du plateau support 16, depuis la face de l'armoire principale 14 où se trouvent les connecteurs 69 vers le deuxième côté 12 du premier chariot 2 et au-delà jusqu'à la portion du plateau support 6 qui est en porte-à-faux (figures 5 et 8). Ces câbles permettent d'alimenter les organes d'instrumentations de l'installation.

On notera que les vannes de drain 36, 42, 54 et 56 sont des vannes trois voies à un orifice d'entrée et deux orifices de sortie, dont l'orifice de sortie drain est soit ouvert soit fermé tandis que l'orifice d'entrée et l'autre orifice de sortie sont toujours ouverts ; et les vannes de débulleur 38, 40, 44 et 46 et les vannes de filtre 62 et 65 sont des vannes trois voies similaires aux vannes d'alimentation 20a et 20b et aux vannes d'entrée 18a à 18e, d'un type différent des vannes de drain (voir ci-après).

On notera également que les éléments décrits plus haut en relation avec le premier chariot 2 se trouvent, sensiblement dans l'empreinte au sol définie par le premier châssis 5. En d'autres termes, ces éléments ne sont pas disposés, à l'exception du collier 76, sur la portion du plateau support 16 qui est en porte-à-faux.

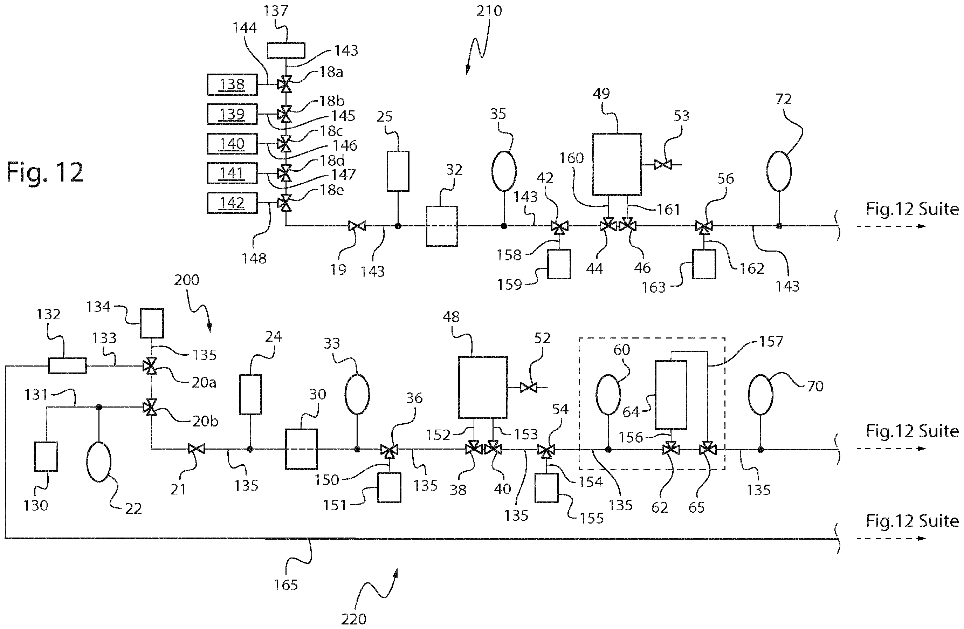

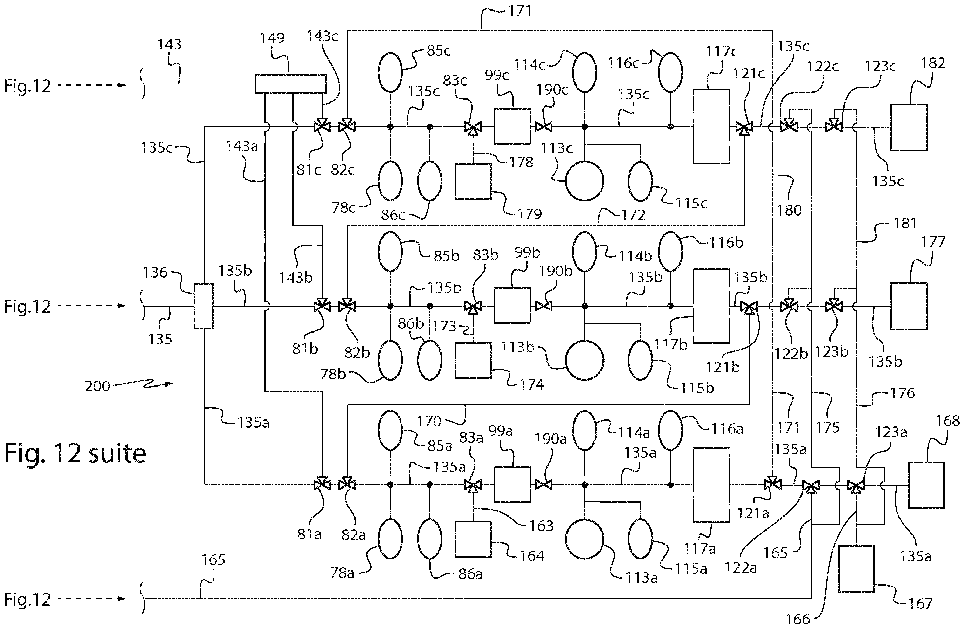

Par ailleurs, ces éléments sont illustrés schématiquement sur la première partie de la figure 12 (planche 9/10 des dessins) et forment principalement des organes d'instrumentation (les vannes et différents capteurs), dont des dispositifs de mesure de paramètres physico-chimiques (les différents capteurs) des liquides circulant dans le circuit de traitement, dans les lignes d'alimentation et additionnelle.

L'installation 1 comporte en outre, montées sur la portion du plateau support 6 en porte-à-faux, trois plateformes dédiées de contrôle et de commande 80a, 80b et 80c qui s'étendent chacune selon une direction verticale par rapport à la direction globalement longitudinale d'extension du premier chariot 2, et qui sont disposées sur le plateau support 16 selon une direction globalement transversale à la direction globalement longitudinale d'extension du premier chariot 2.

En particulier, les trois plateformes 80a, 80b et 80c sont agencées sensiblement en triangle, autour d'une échancrure 79 ménagée à une extrémité de la portion du plateau support 16 qui se trouve en porte-à-faux.

Les plateformes 80a, 80b et 80c étant identiques, une seule sera décrite en détail, à savoir la plateforme 80a, sachant que cette description s'applique à chacune des autres plateformes 80b et 80c, en particulier en référence aux figures 9 à 11.

La plateforme 80a comporte des organes d'instrumentations, dont trois vannes de distribution 81 a, 82a et 83a, chacune pourvue d'un corps et d'une tête prolongeant le corps, et plusieurs dispositifs de mesure dont un capteur de conductivité 78a, un capteur de pH 85a et un capteur de présence d'air 86a.

La plateforme 80a comporte un bloc support 84a, ici de forme sensiblement parallélépipédique, qui s'étend verticalement, dans lequel sont logés les corps des vannes 81 a, 82a et 83a et duquel saillent latéralement les têtes des vanne 81 a, 82a et 83a.

En particulier, les têtes des vannes 81a et 82a saillent d'une première face latérale 87a du bloc support 84a ici tournée vers le premier côté 11 du premier chariot 2, tandis que la tête de la vanne 83a saille d'une deuxième face latérale 88a du bloc support 84a tournée vers le deuxième côté 13 du premier chariot 2.

La plateforme 80a comporte en outre une plaque support 89a fixée sur une troisième face latérale 90a du bloc support 84a, tournée vers l'arrière 10 du premier chariot 2, et présentant un bras 191 a qui saille du bloc support 84a et sur lequel sont montés les capteurs de conductivité 78a, de pH 85a et de présence d'air 86a.

En particulier, le capteur de présence d'air 86a est directement assujetti mécaniquement à une extrémité libre du bras 191 a, tandis que les capteurs de conductivité 78a et de pH 85a sont montés partiellement à emboîtement dans une chambre fluidique 94, laquelle est fixée sur un flasque 93 lui-même assujetti mécaniquement sur le bras 191 a, entre son extrémité libre où est fixé le capteur de présence d'air 86a et la portion de la plaque support 89a fixée sur la troisième face latérale 90a du bloc support 84a.

Cette chambre fluidique 94 fait ici partie intégrante de la ligne d'alimentation du circuit de traitement et présente deux raccords de chambre par exemple mâles pour le raccordement de portions de conduites, l'un des raccords de chambre étant dirigé vers le bloc support 84a et l'autre des raccords de chambre étant dirigé vers le capteur de présence d'air 86a.

La vanne 83a, la vanne 81 a, la vanne 82a, le capteur de conductivité 78a, le capteur de présence d'air 86a et le capteur de pH 85a sont disposés sensiblement les uns au-dessus des autres sur la plateforme dédiée de contrôle et de commande 80a.

Chacune des vannes 81 a, 81 b et 81 c est ici configurée pour être raccordée, sur la ligne d'alimentation, en amont, au quatrième capteur de pression 70 et, en aval, à la vanne 82a, 82b et 82c respective.

Chacune des vannes 81 a, 81 b et 81 c est en outre ici configurée pour être aussi raccordée, sur la ligne additionnelle, en amont, au cinquième capteur de pression 72 et, en aval, à la vanne 82a, 82b et 82c respective.

Chaque vanne 82a, 82b et 82c est ici configurée pour être raccordée, sur la ligne d'alimentation, en amont, à la vanne 81 a, 81 b et 81 c respective et, en aval, à la fois au capteur de pH 85a, 85b et 85c respectif et au capteur de conductivité 78a, 78b et 78c respectif.

Chacun des capteurs de pH 85a, 85b et 85c et capteurs de conductivité 78a, 78b et 78c est donc ici configuré pour être raccordé sur la ligne d'alimentation, en amont, à la vanne 82a, 82b et 82c respective et, en aval, au capteur de présence d'air 86a, 86b et 86c respectif.

Chaque capteur de présence d'air 86a, 86b et 86c est ici configuré pour être raccordé, en amont, à la fois au capteur de pH 85a, 85b et 85c respectif et au capteur de conductivité 78a, 78b et 78c respectif et, en aval, à la vanne 83a, 83b et 83c respective.

Chacune des vannes 83a, 83b et 83c est ici configurée pour être raccordée, sur la ligne d'alimentation, en amont, au capteur de présence d'air 86a, 86b et 86c respectif et, en aval, notamment à un récipient de rebut respectif et à une colonne de chromatographie respective (voir ci-après).

On notera que les vannes de distribution 81 a-c, 82a-c et 83a-c sont ici des vannes similaires aux vannes de filtre 62 et 65, aux vannes d'alimentation 20a et 20b et aux vannes d'entrée 18a à 18e.

Ainsi qu'expliqué plus haut, toutes ces vannes sont pourvues d'un corps de vanne et d'une tête de vanne qui s'étend depuis le corps, laquelle tête de vanne est ici prévue pour recevoir des portions de conduites de la ligne respective (d'alimentation ou additionnelle) où se situe la vanne respective.

En particulier, en référence aux figures 9 à 11, la vanne 81 a est une vannes à trois voies, dont deux entrées et une sortie ou une entrée et deux sorties, pourvue de deux gorges débouchantes 91 et 92 à leurs extrémités, ménagées dans la tête, et configurées pour recevoir des portions de conduites, et ici d'un mécanisme à pincement configuré pour autoriser ou interdire la circulation de liquide dans les portions de conduites reçues dans les deux gorges 91 et 92, par pincement de l'une ou l'autre de ces dernières. Ce mécanisme à pincement est logé dans la tête, entre les deux gorges 91 et 92, et est actionné par un actionneur pneumatique logé dans le corps de vanne. Il s'agit de vannes à pincement multitubes.

Les autres vannes 81b-c, 82a-c, 83a-c, 18a-e, 20a-b, 62 et 65 présentent ici une structure similaire à celle de la vanne 81 a.

En variante, les vannes à trois voies susmentionnées pourraient être d'un autre type que des vannes dites à pincement.

Le deuxième chariot 3 est pourvu d'un deuxième châssis 95 métallique présentant un deuxième cadre inférieur 96 ici sensiblement rectangulaire, monté sur roulettes 97, une planche support posée sur ce deuxième cadre inférieur 96 et une anse de manutention 99, en forme de U inversé, qui saille du deuxième cadre inférieur 96 et qui est prévue pour guider le déplacement du deuxième chariot 3.

On notera que le deuxième cadre inférieur 96 est formé de deux barres longitudinales, appelées aussi longerons, s'étendant selon une direction longitudinale, et de deux barres transversales, appelées aussi traverses, s'étendant selon une direction transversale, ici sensiblement orthogonale à la direction longitudinale. Ici, les traverses et les longerons du deuxième cadre inférieur 96 présentent sensiblement les mêmes dimensions.

L'installation 1 comporte plusieurs colonnes de chromatographie 99a-c, ici au nombre de trois, qui sont posées sur la planche support selon un agencement, ici en triangle, permettant à ces colonnes de s'étendre selon une direction globalement transversale à la direction globalement longitudinale d'extension de l'installation. En d'autres termes, les trois colonnes 99a, 99b et 99c s'étendent dans la longueur de traverses plutôt que dans la longueur des longerons du deuxième cadre inférieur 96.

Ces colonnes 99a-c sont pourvues chacune, sur une face supérieure, d'un raccord d'entrée 100a-c et d'un raccord de sortie associé à une vanne manuelle 101 a-c.

Ces colonnes 99a, 99b et 99c sont ici configurées pour être raccordées, sur la ligne d'alimentation, en amont, respectivement aux vannes de distribution 83a, 83b et 83c au niveau de leur raccord d'entrée respectif 100a-c.

Le troisième chariot 4 est quant à lui pourvu d'un troisième châssis 102 métallique, sensiblement en forme de U inversé, présentant un troisième cadre inférieur 103 ayant une première partie 105 ici sensiblement en forme de C et ouverte sur l'avant 104 du troisième chariot 4, et une seconde partie 106 ici sensiblement rectangulaire et accolée à la première partie 105 sur un premier côté libre 107 (ou extrémité) du troisième chariot 4.

Le troisième chariot 4 est en outre pourvu de montants verticaux 108 assujettis au troisième cadre inférieur 102 et reliés entre eux, sur le haut du troisième châssis 102, via deux barres longitudinales, ou longerons, s'étendant selon une direction longitudinale, et via deux barres transversales, ou traverses, s'étendant selon une direction transversale, ici sensiblement orthogonale à la direction longitudinale.

Le troisième cadre inférieur 102 est lui aussi monté sur quatre roulettes 109 pour faciliter son déplacement.

La forme en U inversé du troisième châssis 102 est prévue pour former un espace d'accueil 110 de sorte à recevoir le deuxième chariot 3 (voir ci-après).

L'installation 1 comporte des supports d'instrumentation additionnels 111 a-c, ici au nombre de trois, disposés en aval des colonnes 99a-c et étant chacun dédiés à une colonne de chromatographie.

Ces supports d'instrumentation additionnels 111 a, 111 b et 111 c sont montés sur le troisième chariot 4, via des consoles de fixation 112a, 112b et 112c respectives qui sont fixées, directement ou indirectement, sur une barre transversale du troisième châssis 102, sur le premier côté libre 107 du troisième chariot 4.

En particulier, ici, les consoles de fixation 112a et 112c sont coudées et directement assujetties mécaniquement sur le troisième châssis 102, tandis que la console de fixation 112b est assujettie mécaniquement sur un tube intermédiaire (non représenté) lui-même fixé directement sur des pattes dédiées des consoles de 112a et 112c.

Ainsi, les supports d'instrumentation additionnels 111 a, 111 b et 111 c sont disposés sur le troisième chariot 4 selon un agencement globalement en triangle et selon la direction transversale.

L'installation 1 comporte plusieurs dispositifs de mesure additionnels portés par chacun de ces supports d'instrumentation additionnels 111 a, 111 b et 111c, dont respectivement des capteurs de conductivité 113a, 113b et 113c, des capteurs de pH 114a, 114b et 114c et des capteurs de rayonnement UV 115a, 115b et 115c, chacun étant monté, par groupe de trois capteurs distincts, sur une console de fixation respective 112a, 112b et 112c.

En particulier, chacun de ces capteurs 113a-c, 114a-c et 115a-c est monté partiellement à emboîtement dans une chambre fluidique 195a, 195b et 195c, sensiblement similaire à celle décrite plus haut, laquelle est fixée sur un flasque ici troué centralement (non représenté) qui est lui-même assujetti mécaniquement la console respective.

Les capteurs de conductivité 113a-c et les capteurs de pH 114a-c sont agencés de manière assez similaire à ceux décrits plus haut au sujet des plateformes 80a-c, c'est-à-dire sensiblement verticalement et respectivement l'un en face de l'autre.

Les capteurs de rayonnement UV 115a-c sont quant à eux agencés sensiblement perpendiculairement aux capteurs de conductivité 113a-c et aux capteurs de pH 114a-c sur chaque support d'instrumentation additionnel 111 a-c respectif, de part et d'autre de la chambre fluidique et du flasque troué, lequel est troué de sorte à permettre aux rayons lumineux de passer depuis un émetteur vers un récepteur en transitant au travers de la chambre fluidique, et ainsi permettre la mesure du rayonnement UV.

La chambre fluidique sur chaque support d'instrumentation additionnel fait ici partie intégrante de la ligne d'alimentation du circuit de traitement et présente deux raccords de chambre par exemple mâles pour le raccordement de portions de conduites.

Chacun des capteurs de conductivité 113a-c, capteurs de pH 114a-c et capteurs de rayonnement UV 115a-c est ici configuré pour être raccordée sur la ligne d'alimentation, en amont, au raccord de sortie associé à la vanne manuelle 101 a, 101 b et 101 c respectif.

L'installation 1 comporte en outre une pluralité d'organes d'instrumentation supplémentaires, dont des capteurs de pression 116a, 116b et 116c, ici sans sécurité, et optionnellement des spectrophotomètres 117a, 117b et 117c.

Les spectrophotomètres 117a, 117b et 117c sont posés sur une tablette 118 montée surélevée par rapport au troisième châssis 102 et assujettie mécaniquement aux barres longitudinales de ce troisième châssis 102, situées sur l'avant 104 et sur l'arrière 120 du troisième chariot 4.

Les capteurs de pression 116a, 116b et 116c sont montés sur cette tablette 118, via des pattes respectives de fixation 119a, 119b et 119c qui sont directement assujetties mécaniquement sur cette tablette 118.

La tablette 118 s'étend globalement selon la direction transversale et les spectrophotomètres 117a-c et capteurs de pression 116a-c sont ainsi disposés selon cette même direction transversale.

Chaque capteur de pression 116a, 116b et 116c est ici configuré pour être raccordé, en amont, à la chambre fluidique d'un support d'instrumentation additionnel 111 a-c respectif pour être connecté à la fois à un capteur de conductivité 113a-c respectif, à un capteur de pH 114a-c respectif et à un capteur de rayonnement UV 115a-c respectif.

Chaque spectrophotomètre 117a, 117b et 117c est ici configuré pour être raccordé sur la ligne d'alimentation, en amont, à un capteur de pression 116a, 116b et 116c respectif.

L'installation 1 comporte en outre des vannes de continuité 121 a, 121 b et 121 c, des vannes de réserve 122a, 122b et 122c, ainsi que des vannes de sortie 123a, 123b et 123c, qui sont montées sur un bloc support 124 en forme d'armoire, disposé sur la seconde partie 106 du troisième cadre inférieur 103 du troisième châssis 102. On notera que les vannes de continuité 121 a, 121 b et 121c et les vannes de réserve 122a, 122b et 122c peuvent aussi être appelées vannes de sortie car elles se trouvent en aval des colonnes.

Les vannes de continuité 121 a sont montées sur une face supérieure du bloc support 124 tandis que les vannes de réserve 122a, 122b et 122c et de sortie 123a, 123b et 123c sont montées sur une face latérale du bloc support 124 sur le premier côté libre 107 du troisième chariot 4.

Ces vannes de continuité 121a-c, de réserve 122a-c et de sortie 123a-c sont ici toutes similaires aux vannes de distribution 81 a-c, 82a-c et 83a-c, aux vannes de filtre 62 et 65, aux vannes d'alimentation 20a et 20b et aux vannes d'entrée 18a à 18e.

En particulier, les vannes de continuité 121a-c, de réserve 122a-c et de sortie 123a-c sont chacune pourvues d'un corps logé dans le bloc support 124 et d'une tête prolongeant le corps et qui saille de la face supérieure ou latérale respective du bloc support 124.

Au surplus, ces vannes de continuité 121a-c, de réserve 122a-c et de sortie 123a-c sont des vannes à pincement multitubes comme celles décrites plus haut.

La vanne de continuité 121a est ici configurée pour être raccordée sur la ligne d'alimentation, en aval, au spectrophotomètre 117a et, en amont, notamment à la vanne de distribution 82c connectée à la colonne 99c de sorte à créer une boucle fluidique depuis la colonne 99a vers la colonne 99c.

La vanne de continuité 121b est ici configurée pour être raccordée sur la ligne d'alimentation, en aval, au spectrophotomètre 117b et, en amont, notamment à la vanne de distribution 82a connectée à la colonne 99a de sorte à créer une boucle fluidique depuis la colonne 99b vers la colonne 99a.

La vanne de continuité 121c est ici configurée pour être raccordée sur la ligne d'alimentation, en aval, au spectrophotomètre 117c et, en amont, notamment à la vanne de distribution 82b connectée à la colonne 99b de sorte à créer une boucle fluidique depuis la colonne 99a vers la colonne 99b.

Chacune des vannes de réserve 122a, 122b et 122c est ici configurée pour être raccordée, en amont, sur la ligne d'alimentation à la vanne de continuité 121 a, 121b et 121c respective et, en aval, notamment sur une ligne de réserve au récipient de réserve, lui-même configuré pour être raccordé à la vanne d'alimentation 20a.

Chacune des vannes de sortie 123a, 123b et 123c est ici configurée pour être raccordée, sur la ligne d'alimentation, en amont, à la vanne de réserve 122a, 122b et 122c respective et, en aval, à un récipient dit de fraction et à récipient de rebut, en fonction de l'état de la vanne respective.

En référence à la figure 2, le troisième chariot 4 est déplacé vers le premier chariot 2 jusqu'à être accolé à ce dernier, par un deuxième côté 125 du troisième chariot 4, opposé à son premier côté libre 107, avec le deuxième côté 13 du premier chariot 2.

Dans cette configuration, le premier châssis 5 du premier chariot 2 et le troisième châssis 102 du troisième chariot 4 sont en butée l'un contre l'autre, avec le haut du troisième châssis 102, formé par les deux barres longitudinales, qui est partiellement emboîté sous la portion du plateau support 16 du premier chariot 2 qui saille du premier châssis 5.

On notera que les premier et troisième chariots 2 et 4 présentent une même profondeur, dans la direction transversale et que l'agencement de l'un par rapport à l'autre est réalisé de sorte que l'espace d'accueil 110 ménagé dans le troisième châssis 102 reste accessible autant par l'avant 104 que par le dessus du troisième chariot 4, notamment via l'échancrure 79 ménagée dans la portion du plateau support 16 qui saille du premier châssis 5.

En référence maintenant à la figure 3, le deuxième chariot 3, déjà préparé avec les trois colonnes 99a-c, est déplacé vers l'ensemble formé par les premier et troisième chariots 2 et 4 puis introduit par l'avant 104 dans l'espace d'accueil 110 ménagé dans le troisième châssis 102 du troisième chariot 4.

Dans cette configuration, le deuxième châssis 95 du deuxième chariot 3 est sensiblement pris en sandwich dans le troisième cadre inférieur 103 du troisième châssis 102 du troisième chariot 4, et les colonnes 99a-c sont disposées sensiblement en-dessous des plateformes de contrôle et de commande 80a-c et des supports d'instrumentation additionnels 111 a-c et sont accessibles depuis le haut de l'installation 1, et en particulier depuis l'échancrure 79 et une partie de l'ouverture ménagée sur le haut du troisième châssis 102, entre les deux barres transversales et la barre longitudinale qui se trouve vers le premier côté libre 107 du troisième chariot 4.

On notera que le deuxième chariot 3 présente une profondeur qui lui permet de ne pas dépasser de l'empreinte au sol, en profondeur, prise par les premier et troisième chariots 2 et 4, sauf à l'avant 107 vers l'avant 107 du troisième chariot 4 où l'anse de manutention 99 reste accessible.

On notera aussi que dans cette configuration, une plateforme de contrôle et de commande 80a-c et un support d'instrumentation additionnel 111a-c sont dédiés à une colonne respective 99a-c.

L'agencement des premier, deuxième et troisième chariots 2 à 4 et des éléments qu'ils comportent permet de fournir une topographie particulièrement compacte, flexible et intuitive du circuit de traitement et de ces éléments qui le composent.

En particulier, il est particulièrement visible que la ligne d'alimentation et la ligne additionnelle s'étendent longitudinalement, sensiblement en parallèle, depuis les vannes d'alimentations 20a-b et les vannes d'entrée 18a-e, jusqu'aux plateformes dédiées de contrôle et de commande 80a-c.

Il est aussi particulièrement visible que la ligne d'alimentation se poursuit ensuite suivant trois bras du circuit de traitement qui s'étendent sur une courte distance longitudinale mais qui cheminent, sensiblement en parallèle, depuis les plateformes dédiées de contrôle et de commande 80a-c jusqu'aux supports d'instrumentation additionnels 111 a-c, en passant par les colonnes 99a-c, et plus généralement jusqu'aux vannes de sortie 123a-c.

Cela permet notamment d'offrir une longueur réduite de tous les chariots 2 à 4, tout en limitant leur profondeur, du fait de la disposition en triangle des trois colonnes 99a-c, mais aussi des plateformes 80a-c et des supports d'instrumentation additionnels 111 a-c.

Cela permet aussi de fournir un circuit de traitement avec des longueurs de conduites réduites et des longueurs sensiblement similaires, sur la première partie du circuit depuis les vannes d'alimentations 20a-b et les vannes d'entrée 18a-e jusqu'aux plateformes dédiées de contrôle et de commande 80a-c, et/ou sur la deuxième partie du circuit depuis plateformes dédiées de contrôle et de commande 80a-c jusqu'aux colonnes 99a-c, et/ou sur la troisième partie du circuit depuis les colonnes 99a-c jusqu'aux vannes de sortie 123a-c ; de sorte à assurer une continuité de traitement, ainsi qu'expliqué plus loin.

On va maintenant décrire le circuit de traitement et un procédé de traitement par chromatographie à l'aide du circuit de traitement fourni par l'installation et illustré schématiquement aux figures 11 et 12.

Sur ces figures, les références 200, 210 et 220 sont respectivement données à la ligne d'alimentation, à la ligne additionnelle et à la ligne de réserve du circuit.

On notera que le terme « conduite » peut être compris dans le présent document comme une portion de tuyauterie, de préférence souple et jetable, reliant des éléments du circuit, cette portion pouvant tout aussi bien comporter un unique tuyau ou au contraire plusieurs tuyaux, présentant éventuellement des diamètres différents.

Le liquide biologique à traiter se trouve initialement dans un récipient dit source ou dans une poche source 130 remplie de liquide en provenance d'un bioréacteur ou en provenance d'un traitement postérieur. Cette poche source 130 est connectée via un raccord à une première conduite 131 qui est raccordée à la vanne d'alimentation 20b sur la ligne d'alimentation 200. Le capteur de présence d'air 22 est aussi connecté sur cette première conduite 131, entre la poche 130 et la vanne 20b.

Le liquide biologique à traiter peut aussi se trouver dans un récipient de réserve 132 remplie de liquide en provenance d'une étape de traitement par chromatographie (voir ci-après). Ce récipient de réserve 132 est connecté via un raccord à une deuxième conduite 133 qui est raccordée à la vanne d'alimentation 20a sur la ligne d'alimentation 200.

Un premier produit tampon d'équilibration se trouve initialement dans un premier récipient d'équilibration 134 qui est connecté via un raccord à une conduite principale 135 de la ligne d'alimentation 200, laquelle conduite principale 135 est raccordée à la vanne d'alimentation 20a et chemine depuis ce premier récipient d'équilibration 134 jusqu'à un premier raccord de dérivation 136, ou distributeur, de l'installation 1 (voir ci-après), où cette conduite principale 135 se subdivise en trois bras 135a, 135b et 135c.

Un second produit tampon d'équilibration se trouve initialement dans un second récipient d'équilibration 137 qui est connecté via un raccord à une conduite additionnelle 143 de la ligne additionnelle 210, laquelle conduite additionnelle 143 est raccordée à la vanne d'entrée 18a et chemine depuis ce second récipient d'équilibration 137 jusqu'à un second raccord de dérivation 149, ou distributeur, de l'installation 1 (voir ci-après), où cette conduite additionnelle 143 se subdivise en trois bras 143a, 143b et 143c.

Un premier produit de lavage se trouve initialement dans un premier récipient de lavage 138 qui est connecté via un raccord à une troisième conduite 144 qui est raccordée à la vanne d'entrée 18a sur la ligne additionnelle 210.

Un second produit de lavage se trouve initialement dans un second récipient de lavage 139 qui est connecté via un raccord à une quatrième conduite 145 qui est raccordée à la vanne d'entrée 18b sur la ligne additionnelle 210.

Un produit d'élution se trouve initialement dans un récipient d'élution 140 qui est connecté via un raccord à une cinquième conduite 146 qui est raccordée à la vanne d'entrée 18c sur la ligne additionnelle 210.

Un produit de nettoyage se trouve initialement dans un récipient de nettoyage 141 qui est connecté via un raccord à une sixième conduite 147 qui est raccordée à la vanne d'entrée 18d sur la ligne additionnelle 210.

Un produit de régénération se trouve initialement dans un récipient de régénération 141 qui est connecté via un raccord à une septième conduite 148 qui est raccordée à la vanne d'entrée 18e sur la ligne additionnelle 210.

Ainsi qu'indiqué ci-avant, il est rappelé que la vanne deux voies 21, le débitmètre 24, la pompe d'alimentation 30, le capteur de pression avec sécurité 33, la première vanne de drain 36, les première et deuxième vannes de débulleur 38 et 40, la troisième vanne de drain 54, les première et deuxième vannes de filtre 62 et 65, et le quatrième capteur de pression 70 sont successivement implantés sur la conduite principale 135 de la ligne d'alimentation 200, depuis la vanne d'alimentation 20b jusqu'au premier raccord de dérivation 136.

La première vanne de drain 36 est ici raccordée à une huitième conduite 150 à laquelle est connecté, via un raccord, un récipient de rebut 151.

La première vanne de débulleur 38 est ici raccordée à une neuvième conduite 152 à laquelle est connectée, via un raccord, l'entrée du premier débulleur 48, et la deuxième vanne de débulleur 40 est ici raccordée à une dixième conduite 153 à laquelle est connectée, via un raccord, la sortie principale du premier débulleur 48.

La troisième vanne de drain 54 est ici raccordée à une onzième conduite 154 à laquelle est connecté, via un raccord, un récipient de rebut 155.

La première vanne de filtre 62 est ici raccordée à une douzième conduite 156 à laquelle est connectée, via un raccord, l'entrée du filtre 64, et la deuxième vanne de filtre 65 est ici raccordée à une treizième conduite 157 à laquelle est connectée, via un raccord, la sortie du filtre 64.

Ainsi qu'indiqué ci-avant, il est rappelé que la vanne deux voies 19, le débitmètre 25, la pompe additionnelle 32, le capteur de pression avec sécurité 35, la deuxième vanne de drain 42, les troisième et quatrième vannes de débulleur 44 et 46, la quatrième vanne de drain 56, et le cinquième capteur de pression 72 sont successivement implantés sur la conduite additionnelle 143 de la ligne additionnelle 210 depuis la vanne d'entrée 18e jusqu'au second raccord de dérivation 149.

La deuxième vanne de drain 42 est ici raccordée à une quatorzième conduite 158 à laquelle est connecté, via un raccord, un récipient de rebut 159.

La troisième vanne de débulleur 44 est ici raccordée à une quinzième conduite 160 à laquelle est connectée, via un raccord, l'entrée du second débulleur 49, et la quatrième vanne de débulleur 46 est ici raccordée à une seizième conduite 161 à laquelle est connectée, via un raccord, la sortie principale du second débulleur 49.

La quatrième vanne de drain 56 est ici raccordée à une dix-septième conduite 162 à laquelle est connecté, via un raccord, un récipient de rebut 163.

Au surplus, les vannes de distribution 81a et 82a, les capteurs de conductivité 78a, de pH 85a et de présence d'air 86a, via la plateforme dédiée de contrôle et de commande 80a, la vanne de distribution 83a, la colonne de chromatographie 99a et une vanne manuelle 190a associée à la colonne 99a en sortie de cette dernière, les capteurs de conductivité 113a, de pH 114a et de rayonnement UV 115a, via le support dédié d'instrumentation additionnel 111a, le capteur de pression 116a, le spectrophotomètre 117a, et les vannes de continuité 121a, de réserve 122a et de sortie 123a sont successivement implantés sur le bras 135a de la conduite principale 135 sur la ligne d'alimentation 200, à partir du premier raccord de dérivation 136.

La vanne de distribution 81a est ici raccordée, en amont, au premier raccord de dérivation 136 et, en aval, au bras 143a de la conduite additionnelle 143.

La vanne de distribution 82a est ici raccordée, en amont, à la vanne de distribution 81a et, en aval, à une première conduite de continuité 170 et aux organes d'instrumentation de la plateforme 80a.

La vanne de distribution 83a est ici raccordée, en aval, aux organes d'instrumentation de la plateforme 80a et, en amont, à une dix-huitième conduite 163 à laquelle est connecté, via un raccord, un récipient de rebut 164, et aux organes d'instrumentation du support additionnel 111a.

La vanne de continuité 121a est ici raccordée, en amont, au spectrophotomètre 117a et, en aval, à une deuxième conduite de continuité 171 et à la vanne de réserve 122a.

La vanne de réserve 122a est ici raccordée, en amont, à la vanne de continuité 121a et, en aval, à une conduite de réserve 165 et à la vanne de sortie 123a. Cette conduite de réserve 165 est ici connectée, via un raccord, au récipient de réserve 132.

La vanne de sortie 123a est ici raccordée, en amont, à la vanne de réserve 122a et, en aval, d'une part à une dix-neuvième conduite 166 à laquelle est connecté, via un raccord, un récipient de fraction 167, et d'autre part à un récipient de rebut 168 via un autre raccord connecté sur le bras 135a de la conduite principale 135.

Les vannes de distribution 81b et 82b, les capteurs de conductivité 78b, de pH 85b et de présence d'air 86b, via la plateforme dédiée de contrôle et de commande 80b, la vanne de distribution 83b, la colonne de chromatographie 99b et une vanne manuelle 190b associée à la colonne 90b en sortie de cette dernière, les capteurs de conductivité 113b, de pH 114b et de rayonnement UV 115b, via le support dédié d'instrumentation additionnel 111b, le capteur de pression 116b, le spectrophotomètre 117b, et les vannes de continuité 121b, de réserve 122b et de sortie 123b sont successivement implantés sur le bras 135b de la conduite principale 135 sur la ligne d'alimentation 200, à partir du premier raccord de dérivation 136.

La vanne de distribution 81b est ici raccordée, en amont, au premier raccord de dérivation 136 et, en aval, au bras 143b de la conduite additionnelle 143.

La vanne de distribution 82b est ici raccordée, en amont, à la vanne de distribution 81b et, en aval, à une troisième conduite de continuité 172 et aux organes d'instrumentation de la plateforme 80b.

La vanne de distribution 83b est ici raccordée, en aval, aux organes d'instrumentation de la plateforme 80b et, en amont, à une vingt-et-une-nième conduite 173 à laquelle est connecté, via un raccord, un récipient de rebut 175, et aux organes d'instrumentation du support additionnel 111b.

La vanne de continuité 121b est ici raccordée, en amont, au spectrophotomètre 117b et, en aval, à la première conduite de continuité 170 et à la vanne de réserve 122b.

La vanne de réserve 122b est ici raccordée, en amont, à la vanne de continuité 121b et, en aval, à une première conduite annexe de réserve 175 et à la vanne de sortie 123b. Cette première conduite annexe de réserve 175 est reliée à la conduite de réserve 165.

La vanne de sortie 123b est ici raccordée, en amont, à la vanne de réserve 122b et, en aval, d'une part à une première conduite annexe de fraction 176 qui est reliée à la conduite de fraction 166, et d'autre part à un récipient de rebut 177 via un raccord connecté sur le bras 135b de la conduite principale 135.

Les vannes de distribution 81c et 82c, les capteurs de conductivité 78c, de pH 85c et de présence d'air 86c, via la plateforme dédiée de contrôle et de commande 80c, la vanne de distribution 83c, la colonne de chromatographie 99c et une vanne manuelle 190c associée à la colonne 99c en sortie de cette dernière, les capteurs de conductivité 113c, de pH 114c et de rayonnement UV 115c, via le support dédié d'instrumentation additionnel 111c, le capteur de pression 116c, le spectrophotomètre 117c, et les vannes de continuité 121c, de réserve 122c et de sortie 123c sont successivement implantés sur le bras 135c de la conduite principale 135 sur la ligne d'alimentation 200, à partir du premier raccord de dérivation 136.

La vanne de distribution 81c est ici raccordée, en amont, au premier raccord de dérivation 136 et, en aval, au bras 143c de la conduite additionnelle 143.

La vanne de distribution 82c est ici raccordée, en amont, à la vanne de distribution 81c et, en aval, à une deuxième conduite de continuité 171 et aux organes d'instrumentation de la plateforme 80c.

La vanne de distribution 83c est ici raccordée, en aval, aux organes d'instrumentation de la plateforme 80c et, en amont, à une vingt-deuxième conduite 178 à laquelle est connecté, via un raccord, un récipient de rebut 179, et aux organes d'instrumentation du support additionnel 111c.

La vanne de continuité 121c est ici raccordée, en amont, au spectrophotomètre 117c et, en aval, à la troisième conduite de continuité 172 et à la vanne de réserve 122c.

La vanne de réserve 122c est ici raccordée, en amont, à la vanne de continuité 121c et, en aval, à une seconde conduite annexe de réserve 180 et à la vanne de sortie 123c. Cette seconde conduite annexe de réserve 180 est reliée à la conduite de réserve 165 via la première conduite annexe de réserve 175.

La vanne de sortie 123c est ici raccordée, en amont, à la vanne de réserve 122c et, en aval, d'une part à une seconde conduite annexe de fraction 181 qui est reliée à la conduite de fraction 166 via la première conduite annexe de fraction 176, et d'autre part à un récipient de rebut 182 via un raccord connecté sur le bras 135c de la conduite principale 135.

Le traitement par chromatographie à l'aide du circuit décrit ci-dessus peut comporter les étapes qui suivent.

Une étape de préparation du circuit est mise en oeuvre, avec une première phase de remplissage.

Pour ce faire, les premier et second récipients d'équilibration 134 et 137 sont raccordés aux conduites principale et additionnelles 135 et 143. Les vannes 18a-e, 19, 20a-b, 21, 36, 38, 40, 42, 44, 46, 54, 56, 62, 65, 81a-c, 82a-c et 83a-c, les pompes 30 et 32, et les raccords de dérivation 136 et 149 sont contrôlés pour faire circuler les liquide d'équilibration en parallèle dans les conduites principale 135 et additionnelle 143, en passant par les premier et second débulleurs 48 et 49 et par le filtre 64 mais en shuntant les récipients de rebut 151, 155, 159 et 163, jusqu'aux vannes 83a-c qui dirigent le liquide dans les récipients de rebut 164, 174 et 179, avant les colonnes 99a-c.

Une deuxième phase de remplissage est mise en oeuvre. Pour ce faire, seul le second récipient d'équilibration 137 est raccordé. Les vannes 18a-e, 19, 42, 44, 46, 81c, 82a-c, 83a-c et 121a-c, la pompe additionnelle 32, et le second raccord de dérivation 149 sont contrôlés pour faire circuler le liquide d'équilibration, successivement, dans la conduite additionnelle 143, en shuntant le second débulleur 49 et les récipients de rebut 159 et 163, dans le bras 135c de la conduite principale 135, au travers de la colonne 99c (en shuntant le récipient de rebut 179) et dans la troisième conduite de continuité 172 jusqu'à la vanne 83b qui dirige le liquide dans le récipient de rebut 174 avant la colonne 99b.

Lorsque le capteur de présence d'air 86c ne détecte plus d'air, la vanne 83b est contrôlée pour shunter le récipient de rebut 174 et pour que le liquide chemine au-delà du chemin précédent, à travers la colonne 99b et dans la première conduite de continuité 170 jusqu'à la vanne 83a qui dirige le liquide dans le récipient de rebut 164 avant la colonne 99a.

Lorsque le capteur de présence d'air 86a ne détecte plus d'air, la vanne 83a est contrôlée pour shunter le récipient de rebut 164 et pour que le liquide chemine au-delà du chemin précédent, à travers la colonne 99a et dans la deuxième conduite de continuité 171.

On notera que les deux phases de remplissage peuvent être mises en oeuvre avec un autre produit que le produit tapon d'équilibration.

Une phase d'équilibration successivement de chacune des colonnes 99a-c est ensuite mise en oeuvre.

Pour ce faire, seul le second récipient d'équilibration 137 est raccordé. Les vannes 18a-e, 19, 42, 44, 46, 81 c, 82c, 83c, 121c, 122c et 123c, la pompe additionnelle 32, et le second raccord de dérivation 149 sont contrôlés pour faire circuler le liquide d'équilibration dans la conduite additionnelle 143, en shuntant le second débulleur 49 et les récipients de rebut 159 et 163, dans le bras 135c de la conduite principale 135, au travers de la colonne 99c (en shuntant le récipient de rebut 179) jusqu'à la vanne 123c qui dirige le liquide dans le récipient de rebut 182 ; jusqu'à ce que les valeurs mesurées de pH et de conductivité par les capteurs 113c et 114c après la colonne 99c soient identiques aux valeurs mesurées de pH et de conductivité par les capteurs 78c et 85c, avant la colonne 99c.

Ensuite, le second récipient d'équilibration 137 est raccordé, les vannes 18a-e, 19, 42, 44, 46, 81 b, 82b, 83b, 121b, 122b et 123b, la pompe additionnelle 32, et le second raccord de dérivation 149 sont contrôlés pour faire circuler le liquide d'équilibration dans la conduite additionnelle 143, en shuntant le second débulleur 49 et les récipients de rebut 159 et 163, dans le bras 135b de la conduite principale 135, au travers de la colonne 99b (en shuntant le récipient de rebut 174) jusqu'à la vanne 123b qui dirige le liquide dans le récipient de rebut 177; jusqu'à ce que les valeurs mesurées de pH et de conductivité par les capteurs 113b et 114b après la colonne 99b soient identiques aux valeurs mesurées de pH et de conductivité par les capteurs 78b et 85b, avant la colonne 99b.

Ensuite, le second récipient d'équilibration 137 est raccordé, les vannes 18a-e, 19, 42, 44, 46, 81a, 82a, 83a, 121a, 122a et 123a, la pompe additionnelle 32, et le second raccord de dérivation 149 sont contrôlés pour faire circuler le liquide d'équilibration dans la conduite additionnelle 143, en shuntant le second débulleur 49 et les récipients de rebut 159 et 163, dans le bras 135a de la conduite principale 135, au travers de la colonne 99a (en shuntant le récipient de rebut 164) jusqu'à la vanne 123a qui dirige le liquide dans le récipient de rebut 168 ; jusqu'à ce que les valeurs mesurées de pH et de conductivité par les capteurs 113a et 114a après la colonne 99a soient identiques aux valeurs mesurées de pH et de conductivité par les capteurs 78a et 85a, avant la colonne 99a.

Une phase de premier chargement peut être effectuée. Pour ce faire, le récipient de produit 130 (ou poche souple) est raccordé à la première conduite 131. Les vannes 20b, 21, 36, 38, 40, 54, 62, 65, 81c, 82c, 83c, 121c, 82b, 83b, 121b, 122b et 123b, le premier raccord de dérivation 136 et la pompe d'alimentation 30 sont contrôlés pour faire circuler le liquide biologique à traiter, successivement, dans la conduite principale 135 en passant par le premier débulleur 48, par le filtre 64, en shuntant les récipients de rebut 151 et 155, puis dans le bras 135c en shuntant le récipient de rebut 179 et en traversant la colonne 99c, puis dans la troisième conduite de continuité 172, puis dans le bras 135b, en shuntant le récipient de rebut 174, en traversant la colonne 99b, en shuntant les récipients de réserve 132 et de fraction 167, jusqu'à la vanne 123b qui dirige le liquide traité dans le récipient de rebut 177.

Une nouvelle phase de chargement peut être effectuée, en particulier lorsque la colonne 99c est dite chargée. Pour ce faire, le récipient de produit 130 (ou poche souple) est toujours raccordé à la première conduite 131. Les vannes 20b, 21, 36, 38, 40, 54, 62, 65, 81b, 82b, 83b, 121b, 82a, 83a, 121a, 122a et 123a, le premier raccord de dérivation 136 et la pompe d'alimentation 30 sont contrôlés pour faire circuler le liquide biologique à traiter, successivement, dans la conduite principale 135 en passant par le premier débulleur 48, par le filtre 64, en shuntant les récipients de rebut 151 et 155, puis dans le bras 135b en shuntant le récipient de rebut 174 et en traversant la colonne 99b, puis dans la première conduite de continuité 170, puis dans le bras 135a, en shuntant le récipient de rebut 164, en traversant la colonne 99a, en shuntant les récipients de réserve 132 et de fraction 167, jusqu'à la vanne 123a qui dirige le liquide traité dans le récipient de rebut 168.

En parallèle de cette nouvelle phase de chargement, une phase de récupération et préparation de la colonne 99c peut être effectuée. Pour ce faire, les premier et second récipients de lavage 138 et 139, le récipient d'élution 140, le récipient de nettoyage 141 et le récipient de régénération 142 sont raccordés aux conduites respectives 144 à 148. Les vannes 18a-e, 19, 42, 44, 46, 56, 81c, 82c, 83c, 121c, 122c et 123c, le second raccord de dérivation 149 et la pompe additionnelle 32 sont contrôlés pour faire circuler :
- le liquide de premier nettoyage dans la conduite additionnelle 143, en passant par le second débulleur 49 et en shuntant les récipients de rebut 159 et 163, puis dans le bras 135c, en shuntant le récipient de rebut 179, en traversant la colonne 99c, soit jusqu'à la vanne de réserve 122c qui dirige le liquide de premier nettoyage vers le récipient de réserve 132 en vue d'un retraitement, soit jusqu'à la vanne de sortie 123c (en shuntant le récipient de fraction 167) qui dirige le liquide de premier nettoyage vers le récipient de rebut 182 ; puis
- optionnellement, le liquide de second nettoyage dans la conduite additionnelle 143, en passant par le second débulleur 49 et en shuntant les récipients de rebut 159 et 163, puis dans le bras 135c, en shuntant le récipient de rebut 179, en traversant la colonne 99c, jusqu'à la vanne de sortie 123c (en shuntant le récipient de réserve 132 et le récipient de fraction 167) qui dirige le liquide de second nettoyage vers le récipient de rebut 182 ; puis
- le liquide d'élution dans la conduite additionnelle 143, en passant par le second débulleur 49 et en shuntant les récipients de rebut 159 et 163, puis dans le bras 135c, en shuntant le récipient de rebut 179, en traversant la colonne 99c, jusqu'à la vanne de sortie 123c qui dirige le liquide d'élution vers le récipient de fraction 167, en shuntant le récipient de réserve 132, lorsque le pic d'élution est détecté par le biais du capteur UV 115c ; puis
- le liquide de nettoyage dans la conduite additionnelle 143, en passant par le second débulleur 49 et en shuntant les récipients de rebut 159 et 163, puis dans le bras 135c, en shuntant le récipient de rebut 179, en traversant la colonne 99c, jusqu'à la vanne de sortie 123c qui dirige le liquide de nettoyage vers le récipient de rebut 182 (en shuntant le récipient de réserve 132 et le récipient de fraction 167) ; puis
- le liquide de régénération dans la conduite additionnelle 143, en passant par le second débulleur 49 et en shuntant les récipients de rebut 159 et 163, puis dans le bras 135c, en shuntant le récipient de rebut 179, en traversant la colonne 99c, jusqu'à la vanne de sortie 123c qui dirige le liquide de régénération vers le récipient de rebut 182 (en shuntant le récipient de réserve 132 et le récipient de fraction 167).

Une nouvelle phase de chargement peut être effectuée, en particulier lorsque la colonne 99b est dite chargée. Pour ce faire, le récipient de produit 130 (ou poche souple) est toujours raccordé à la première conduite 131. Les vannes 20b, 21, 36, 38, 40, 54, 62, 65, 81a, 82a, 83a, 121 a, 82c, 83c, 121c, 122c et 123c, le premier raccord de dérivation 136 et la pompe d'alimentation 30 sont contrôlés pour faire circuler le liquide biologique à traiter, successivement, dans la conduite principale 135 en passant par le premier débulleur 48, par le filtre 64, en shuntant les récipients de rebut 151 et 155, puis dans le bras 135a en shuntant le récipient de rebut 164 et en traversant la colonne 99a, puis dans la deuxième conduite de continuité 171, puis dans le bras 135c, en shuntant le récipient de rebut 179, en traversant la colonne 99c, en shuntant les récipients de réserve 132 et de fraction 167, jusqu'à la vanne 123c qui dirige le liquide traité dans le récipient de rebut 182.

En parallèle de cette nouvelle phase de chargement, une phase de récupération et préparation de la colonne 99b peut être effectuée. Pour ce faire, les premier et second récipients de lavage 138 et 139, le récipient d'élution 140, le récipient de nettoyage 141 et le récipient de régénération 142 sont raccordés aux conduites respectives 144 à 148. Les vannes 18a-e, 19, 42, 44, 46, 56, 81b, 82b, 83b, 121b, 122b et 123b, le second raccord de dérivation 149 et la pompe additionnelle 32 sont contrôlés pour faire circuler :
- le liquide de premier nettoyage dans la conduite additionnelle 143, en passant par le second débulleur 49 et en shuntant les récipients de rebut 159 et 163, puis dans le bras 135b, en shuntant le récipient de rebut 174, en traversant la colonne 99b, soit jusqu'à la vanne de réserve 122b qui dirige le liquide de premier nettoyage vers le récipient de réserve 132 en vue d'un retraitement, soit jusqu'à la vanne de sortie 123b (en shuntant le récipient de fraction 167) qui dirige le liquide de premier nettoyage vers le récipient de rebut 177 ; puis
- optionnellement, le liquide de second nettoyage dans la conduite additionnelle 143, en passant par le second débulleur 49 et en shuntant les récipients de rebut 159 et 163, puis dans le bras 135b, en shuntant le récipient de rebut 174, en traversant la colonne 99b, jusqu'à la vanne de sortie 123b (en shuntant le récipient de réserve 132 et le récipient de fraction 167) qui dirige le liquide de second nettoyage vers le récipient de rebut 177 ; puis
- le liquide d'élution dans la conduite additionnelle 143, en passant par le second débulleur 49 et en shuntant les récipients de rebut 159 et 163, puis dans le bras 135b, en shuntant le récipient de rebut 174, en traversant la colonne 99b, jusqu'à la vanne de sortie 123b qui dirige le liquide d'élution vers le récipient de fraction 167, en shuntant le récipient de réserve 132, lorsque le pic d'élution est détecté par le biais du capteur UV 115b ; puis
- le liquide de nettoyage dans la conduite additionnelle 143, en passant par le second débulleur 49 et en shuntant les récipients de rebut 159 et 163, puis dans le bras 135b, en shuntant le récipient de rebut 174, en traversant la colonne 99b, jusqu'à la vanne de sortie 123b qui dirige le liquide de nettoyage vers le récipient de rebut 177 (en shuntant le récipient de réserve 132 et le récipient de fraction 167) ; puis
- le liquide de régénération dans la conduite additionnelle 143, en passant par le second débulleur 49 et en shuntant les récipients de rebut 159 et 163, puis dans le bras 135b, en shuntant le récipient de rebut 174, en traversant la colonne 99b, jusqu'à la vanne de sortie 123b qui dirige le liquide de régénération vers le récipient de rebut 177 (en shuntant le récipient de réserve 132 et le récipient de fraction 167).

Une nouvelle phase de chargement peut être effectuée, en particulier lorsque la colonne 99a est dite chargée. Cette nouvelle phase de chargement est identique à la phase précédente dite de premier chargement, avec le liquide biologique qui traverse les colonnes 99c puis 99b. Alternativement, cette nouvelle phase de chargement peut se faire grâce au raccordement du récipient de réserve 132 à la vanne d'alimentation 20a plutôt que le raccordement du récipient de produit 130, de sorte à traiter le premier produit de nettoyage récupéré.

En parallèle de cette nouvelle phase de chargement, une phase de récupération et préparation de la colonne 99a peut être effectuée. Pour ce faire, les premier et second récipients de lavage 138 et 139, le récipient d'élution 140, le récipient de nettoyage 141 et le récipient de régénération 142 sont raccordés aux conduites respectives 144 à 148. Les vannes 18a-e, 19, 42, 44, 46, 56, 81a, 82a, 83a, 121a, 122a et 123a, le second raccord de dérivation 149 et la pompe additionnelle 32 sont contrôlés pour faire circuler :
- le liquide de premier nettoyage dans la conduite additionnelle 143, en passant par le second débulleur 49 et en shuntant les récipients de rebut 159 et 163, puis dans le bras 135a, en shuntant le récipient de rebut 164, en traversant la colonne 99a, soit jusqu'à la vanne de réserve 122a qui dirige le liquide de premier nettoyage vers le récipient de réserve 132 en vue d'un retraitement, soit jusqu'à la vanne de sortie 123a (en shuntant le récipient de fraction 167) qui dirige le liquide de premier nettoyage vers le récipient de rebut 168 ; puis
- optionnellement, le liquide de second nettoyage dans la conduite additionnelle 143, en passant par le second débulleur 49 et en shuntant les récipients de rebut 159 et 163, puis dans le bras 135a, en shuntant le récipient de rebut 164, en traversant la colonne 99a, jusqu'à la vanne de sortie 123a (en shuntant le récipient de réserve 132 et le récipient de fraction 167) qui dirige le liquide de second nettoyage vers le récipient de rebut 168 ; puis
- le liquide d'élution dans la conduite additionnelle 143, en passant par le second débulleur 49 et en shuntant les récipients de rebut 159 et 163, puis dans le bras 135a, en shuntant le récipient de rebut 164, en traversant la colonne 99a, jusqu'à la vanne de sortie 123a qui dirige le liquide d'élution vers le récipient de fraction 167, en shuntant le récipient de réserve 132, lorsque le pic d'élution est détecté par le biais du capteur UV 115a ; puis
- le liquide de nettoyage dans la conduite additionnelle 143, en passant par le second débulleur 49 et en shuntant les récipients de rebut 159 et 163, puis dans le bras 135a, en shuntant le récipient de rebut 164, en traversant la colonne 99a, jusqu'à la vanne de sortie 123a qui dirige le liquide de nettoyage vers le récipient de rebut 168 (en shuntant le récipient de réserve 132 et le récipient de fraction 167) ; puis
- le liquide de régénération dans la conduite additionnelle 143, en passant par le second débulleur 49 et en shuntant les récipients de rebut 159 et 163, puis dans le bras 135a, en shuntant le récipient de rebut 164, en traversant la colonne 99a, jusqu'à la vanne de sortie 123a qui dirige le liquide de régénération vers le récipient de rebut 168 (en shuntant le récipient de réserve 132 et le récipient de fraction 167).

Le traitement peut se poursuivre, de manière continue, en mettant en oeuvre à nouveau les mêmes phases que celles décrites ci-dessus.

Bien entendu, ici, le traitement a débuté avec la mise en série des colonnes 99c et 99b mais il pourrait débuter avec la mise en série par exemple des colonnes 99b et 99a ou 99a et 99c.

Au surplus, il est à noter que les vannes manuelles 190a-c sont par défaut ouverte pendant tout le traitement de chromatographie, dès lors que les colonnes 99a-c sont raccordées sur le circuit. Elles sont fermées lorsque le traitement est terminé et que les colonnes 99a-c sont retirées du circuit de l'installation.

Dans des variantes non illustrées :
- l'installation peut comporter plus que trois colonnes de chromatographie, voire seulement deux colonnes de chromatographie ;
- les colonnes pourraient être disposées en cercle ou en losange plutôt qu'en triangle ;
- l'installation peut comporter plus ou moins de plateformes de contrôle et de commande, voire de supports d'instrumentation additionnels, en fonction du nombre de colonnes de chromatographie ;
- l'installation peut comporter plus ou moins d'organes d'instrumentation montés sur chaque plateforme de contrôle et de commande ;
- l'installation peut être dépourvue de spectrophotomètre, ou ne comporter qu'un seul spectrophotomètre, situé comme sur la tablette du troisième chariot ou bien située sur un support annexe, non logé sur le troisième chariot, et configuré pour être raccordée sur la dix-neuvième conduite 166 juste avant le récipient de fraction 167 ;
- les supports d'instrumentation additionnels et les organes d'instrumentation qu'ils comportent pourraient être montés aussi, par exemple selon l'orientation verticale, sur les plateformes de contrôle et de commande, tout en conservant leurs fonctions d'instrumentation dites post-colonnes ; de sorte que les plateformes de contrôle et de commande fourniraient à la fois des fonctions d'instrumentation dites pré-colonnes et post-colonnes ;
- l'installation pourrait être pourvue de seulement deux chariots, les premier et deuxième chariots étant combinés, ou les premier et troisième chariots étant combinés ou encore les deuxième et troisième chariots étant combinés, voire seulement un chariot, voire au contraire plus de trois chariots ;
- l'agencement des organes d'instrumentation en amont et en aval des plateformes verticales pourrait être différent sur l'armoire principale, sur le plateau support et sur le troisième châssis ; et
- les phases mises en oeuvre dans le traitement par chromatographie peuvent être différentes de celles décrites plus haut, en particulier en vue de la récupération et de la préparation de la colonne chargée.

On rappelle plus généralement que l'invention ne se limite pas aux exemples décrits et représentés.

## Revendications

1. Installation de traitement de liquide biologique par chromatographie, s'étendant globalement selon une direction longitudinale et comportant :
- au moins une vanne d'alimentation (20b) en liquide biologique à traiter, configurée pour être raccordée au moins à un récipient d'alimentation (130) en liquide biologique ;
- au moins une pompe d'alimentation (30) disposée en aval de ladite au moins une vanne d'alimentation et raccordée à cette dernière ;
- une pluralité d'organes d'instrumentation disposés en aval de ladite au moins une pompe d'alimentation, dont au moins une vanne de distribution (81a-c, 82a-c, 83a-c) et au moins un dispositif de mesure (78a-c, 85a-c, 86a-c) d'un paramètre physico-chimique du liquide biologique, et qui sont raccordés à ladite au moins une pompe d'alimentation ;
- au moins une colonne de chromatographie (99a-c) disposée en aval de ladite pluralité d'organes d'instrumentation et directement associée et raccordée à au moins certains d'entre eux, et configurée pour être alimentée en liquide biologique par ladite au moins une pompe d'alimentation ; et
- une pluralité de conduites à usage unique configurées pour être connectées à ladite au moins une vanne d'alimentation, à ladite au moins une pompe d'alimentation, à ladite pluralité d'organes d'instrumentation et à ladite au moins une colonne de chromatographie, de sorte à former au moins une ligne d'alimentation (200) de liquide biologique à traiter d'un circuit de traitement de ladite installation ;
**caractérisée en ce que** lesdits organes d'instrumentation associés à ladite au moins une colonne de chromatographie sont montés chacun sur au moins une plateforme dédiée de contrôle et de commande (80a-c), qui s'étend selon une direction verticale par rapport à la direction globalement longitudinale d'extension de ladite installation, et sont disposés sensiblement les uns au-dessus des autres sur ladite plateforme dédiée de contrôle et de commande.

2. Installation selon la revendication 1, **caractérisée en ce que** ladite au moins une plateforme de contrôle et de commande (80a-c) comporte plusieurs vannes de distribution (81a-c, 82a-c, 83a-c) et un ou plusieurs dispositifs de mesure choisis parmi un capteur de conductivité (78a-c) et/ou un capteur de pH (85a-c) et/ou un capteur de présence d'air (86a-c).

3. Installation selon la revendication 2, **caractérisée en ce que** lesdites vannes de distribution (81a-c, 82a-c, 83a-c) sont pourvues chacune d'un corps de vanne et d'une tête de vanne qui s'étend depuis ledit corps et qui est prévue pour recevoir au moins des portions de conduites de ladite ligne d'alimentation, et ladite au moins une plateforme de contrôle et de commande (80a-c) comporte un bloc support (84a-c) qui s'étend verticalement, dans lequel sont logés lesdits corps de vanne et desquels saillent latéralement lesdites têtes de vanne, ainsi qu'une plaque support (89a-c) fixée sur ledit bloc support et présentant un bras (191a-c) qui saille dudit bloc support et sur lequel est monté ledit au moins un dispositif de mesure (78a-c, 85a-c, 86a-c).

4. Installation selon l'une quelconque des revendications 1 à 3, **caractérisée en ce qu'**elle comporte plusieurs colonnes de chromatographie (99a-c) disposées en aval de ladite au moins une plateforme de contrôle et de commande (80a-c), et qui s'étendent selon une direction globalement transversale à ladite direction globalement longitudinale d'extension de ladite l'installation.

5. Installation selon la revendication 4, **caractérisée en ce qu'**elle comporte plusieurs plateformes dédiées de contrôle et de commande (80a-c), chacune étant individuellement associée à une desdites colonnes de chromatographie (99a-c), lesdites plateformes dédiées de contrôle et de commande étant disposées selon la même direction globalement transversale que lesdites colonnes de chromatographie.

6. Installation selon la revendication 5, **caractérisée en ce que** lesdites plateformes de contrôle et de commande (80a-c) sont montées sur un premier chariot (2) et lesdites colonnes de chromatographie (99a-c) sont montées sur un deuxième chariot (3) configuré pour être accolé et/ou partiellement emboîté avec ledit premier chariot.

7. Installation selon la revendication 6, **caractérisée en ce que** ledit premier chariot (2) comporte un premier châssis (5), au moins une armoire de distribution électrique et pneumatique (14) montée sur ledit châssis et sur laquelle est disposée ladite au moins une vanne d'alimentation (20b), au moins un espace d'accueil (59) formé dans ledit premier châssis et prévu pour recevoir des récipients de récupération de liquides, et au moins un plateau support (16) monté saillant sur ledit premier châssis et sur lequel sont disposées ladite au moins une pompe d'alimentation (30) et lesdites plateformes de contrôle et de commande (80a-c), et ledit deuxième chariot (3) comporte un deuxième châssis (95) pourvu d'une planche support prévue pour recevoir lesdites colonnes de chromatographie (99a-c) et configurée pour venir au moins partiellement en emboîtement sous ledit plateau support (16) dudit premier chariot.

8. Installation selon l'une des revendications 1 à 7, **caractérisée en ce qu'**elle comporte en outre des supports d'instrumentation additionnels (11a-c) disposés en aval desdites colonnes de chromatographie (99a-c) et sur lesquels sont montés un ou plusieurs dispositifs de mesure additionnels choisis parmi un capteur de conductivité (113a-c), et/ou un capteur de pH (114a-c) et/ou un capteur de rayonnement UV (115a-c).

9. Installation selon les revendications 7 et 8, **caractérisée en ce que** lesdits supports d'instrumentation additionnels (111a-c) sont montés sur un troisième chariot (4) configuré pour être accolé et/ou partiellement emboîté avec ledit premier chariot (2) et/ou avec ledit deuxième chariot (3).

10. Installation selon la revendication 9, **caractérisée en ce qu'**elle comporte en outre une pluralité d'organes d'instrumentation supplémentaires disposés en aval desdites colonnes de chromatographie (99a-c), dont au moins une vanne de sortie (121a-c, 122a-c, 123a-c) et au moins un dispositif de mesure supplémentaire choisis parmi un capteur de pression (116a-c) et/ou un spectrophotomètre (117a-c), lesdits organes d'instrumentation supplémentaires étant montés sur ledit troisième chariot -4), en aval desdits supports d'instrumentation additionnels (111a-c).

11. Installation selon l'une des revendications 9 ou 10, **caractérisée en ce que** ledit troisième chariot (4) comporte un troisième châssis (102), sensiblement en forme de U inversé, sur lequel sont montés au moins lesdits supports d'instrumentation additionnels (111a-c) à une extrémité libre dudit troisième châssis, et qui est configuré pour être accolé audit premier châssis (5) dudit premier chariot (2) par une extrémité opposée à son extrémité libre, et pour recevoir à emboîtement, dans un espace (110) formé par le U inversé entre son extrémité opposée et son extrémité libre, ledit deuxième châssis (95) dudit deuxième chariot (3) pourvu desdites colonnes de chromatographie (99a-c).

12. Installation selon l'une quelconque des revendications 1 à 11, **caractérisée en ce que** ladite au moins une vanne d'alimentation et/ou ladite au moins une vanne de distribution sont des vannes à trois voies, dont deux entrées et une sortie ou une entrée et deux sorties, et de préférence une vanne pourvue d'une tête présentant deux gorges de réception (91, 92) de portions de dites conduites, et d'un mécanisme à pincement configuré pour autoriser ou interdire la circulation dudit liquide biologique dans lesdites portions de dites conduites reçues dans les deux dites gorges (91, 92).

13. Installation selon l'une quelconque des revendications 1 à 12, **caractérisée en ce qu'**elle comporte en outre un récipient de réserve (132) disposé en aval de ladite au moins une colonne de chromatographie (99a-c) et raccordé à cette dernière, ledit récipient de réserve étant prévu pour recevoir un produit tampon utilisé pour le nettoyage de ladite au moins une colonne de chromatographie après traitement dudit liquide biologique, et une autre vanne d'alimentation (20a) disposée en aval dudit récipient de réserve et en amont de ladite au moins une pompe d'alimentation (30) et raccordée à ceux-ci, ladite autre vanne d'alimentation (20a) étant configurée pour réintroduire, via ladite au moins une pompe d'alimentation, ledit produit tampon sur ladite ligne d'alimentation (200) en tant que liquide à traiter dans ladite au moins une colonne de chromatographie (99a-c).

14. Installation selon l'une quelconque des revendications 1 à 13, **caractérisée en ce qu'**elle comporte en outre une pluralité de vannes d'entrée (18a-e) configurées pour être raccordées à des récipient de produits (138-142) dits tampon et pour alimenter ledit circuit de traitement en vue de la préparation et/ou du nettoyage et/ou de l'élution et/ou de la régénération de ladite au moins une colonne de chromatographie (99a-c), et au moins une pompe additionnelle (32) disposée en aval desdites vannes d'entrée et en amont de ladite au moins une colonne de chromatographie et raccordée à ces dernières ; des conduites de ladite pluralité de conduites à usage unique étant configurées pour être connectées auxdites vannes d'entrée et à ladite pompe additionnelle, de sorte à former au moins une ligne additionnelle (210), qui s'étend sensiblement longitudinalement et en parallèle de ladite ligne d'alimentation (200), depuis respectivement lesdites vannes d'entrée (18a-e) et ladite au moins une vanne d'alimentation (20b), vers ladite au moins une plateforme dédiée de contrôle et de commande 80a-c).

15. Installation selon la revendication 14, **caractérisée en ce qu'**elle comporte en outre d'autres organes d'instrumentation, dont, sur ladite ligne d'alimentation (200) :
- un capteur de présence produit (22) disposé en amont de ladite au moins une vanne d'alimentation (20b) ; et/ou
- une vanne deux voies d'isolation (21) et/ou un débitmètre (24) disposés entre ladite au moins une vanne d'alimentation et ladite au moins une pompe d'alimentation (30) ; et/ou
- un capteur de pression (33) avec ou sans sécurité, et/ou au moins une vanne de drain (36, 54), et/ou au moins deux vannes de débulleur (38, 40) et un débulleur (48) raccordé à chacune de ces vannes de débulleur et/ou au moins une vanne de filtre (62, 65) et un filtre (64) raccordé à ladite au moins une vanne de filtre, disposés entre ladite au moins une pompe d'alimentation et ladite au moins une plateforme de contrôle et de commande (80a-c) ; et/ou
sur ladite ligne additionnelle (210) :
- une vanne deux voies d'isolation (19) et/ou un débitmètre (25) disposés entre lesdites vannes d'entrée (18a-e) et ladite au moins une pompe additionnelle (32) ; et/ou
- au moins un capteur de pression (35, 72) avec ou sans sécurité, et/ou au moins une vanne de drain (42, 56), et/ou au moins deux vannes de débulleur (44, 46) et un débulleur (49) raccordé à chacune de ces vannes de débulleur, disposés entre ladite pompe additionnelle et ladite au moins une plateforme de contrôle et de commande.
